# EUROPEAN PATENT APPLICATION

(11) **EP 4 272 778 A1**
(43) Date of publication of application: **08.11.2023**
(21) Application number: 20967880.4
(22) Date of filing: 31.12.2020
(51) Int. Cl.: A61M 5/142, A61M 5/168, A61M 39/28, A61M 5/172, A61M 5/175

(54) **INFUSION PUMP AND INFUSION PUMP CONTROL METHOD**

(71) Applicant: Shenzhen Mindray Scientific Co., Ltd., Shenzhen, Guangdong 518057 (CN)
(72) Inventor: ZHANG, Peng, Shenzhen, Guangdong 518107 (CN); ZUO, Pengfei, Shenzhen, Guangdong 518107 (CN); PENG, Mingxu, Shenzhen, Guangdong 518107 (CN); CHEN, Guiyang, Shenzhen, Guangdong 518107 (CN)
(74) Representative: KIPA AB
(86) International application number: PCT/CN2020/142447
(87) International publication number: WO 2022/141542

(57) **Abstract**

Embodiments of the present application provide an infusion pump and an infusion pump control method. The infusion pump comprises a pump body, a pump door, and a liquid stopping clamp driving module. The pump body comprises a face frame. The face frame is provided with a liquid stopping clamp mounting position and an infusion tube mounting position. The pump door is movably connected to the pump body. The liquid stopping clamp driving module comprises a movable mechanism. When being loaded on the liquid stopping clamp mounting position, a liquid stopping clamp abuts against the movable mechanism in a liquid stopping working position, so that the liquid stopping clamp is closed to clamp the infusion tube so as to cut off a liquid circulation path in the infusion tube; when the movable mechanism switches from the liquid stopping working position to a conduction working position under the action of the driving force, the liquid stopping clamp unclamps the infusion tube to conduct the liquid circulation path in the infusion tube; when the movable mechanism switches from the conduction working position to the liquid stopping working position under the action of the driving force, the liquid stopping clamp clamps the infusion tube to cut off the liquid circulation path in the infusion tube. The infusion pump of the embodiments of the present application facilitates liquid stopping and is compact in structure.

## Description

### TECHNICAL FIELD

The disclosure relates to the technical field of medical device, and more particularly to an infusion pump and a control method for an infusion pump.

### BACKGROUND

As a main infusion product for clinical infusion, it is crucial to accurately stop infusion for the sake of infusion safety. A typical infusion pump is provided with a liquid-stopping component. When the infusion tube is loaded at the infusion pump, the liquid-stopping component is configured to disconnect a path for a liquid flow inside the infusion tube before or after the infusion is stopped. When the infusion is started, the path for the liquid flow inside the infusion tube is connected. However, this liquid-stopping component cannot disconnect the infusion tube when the user loads or takes out the infusion tube. The user also needs to manually close a liquid-stopping roller to prevent the liquid from dripping and polluting the environment.

In order to improve the accuracy of infusion, another type of infusion pump is also provided in the industry. An additional liquid-stopping clamp structure is arranged next to the liquid-stopping component, which liquid-stopping clamp structure can be combined with the infusion tube and installed at the infusion pump. However, before starting the infusion, this type of infusion pump needs to connect the original liquid-stopping component, as well as the liquid-stopping clamp structure at the infusion tube, which is relatively cumbersome in overall control steps. Some operation steps also require manual operation by the user, such as manually closing the liquid-stopping clamp structure during moving the infusion tube. In addition, this type of infusion pump requires a large installation space in a length direction of the infusion pump due to the parallel arrangement of the liquid-stopping clamp and the movable mechanism, which makes the overall structure of the infusion pump relatively large, thereby affecting the miniaturization of the infusion pump.

### SUMMARY

In view of this, embodiments of this disclosure expect to provide an infusion pump with simple liquid stopping control and more compact overall structure, and a control method for an infusion pump.

In order to achieve the above purpose, a first aspect of an embodiment of this disclosure provides an infusion pump, which is used in cooperation with a liquid-stopping clamp which is sleeved outside an infusion tube; wherein the infusion pump includes:
a main body, which includes a surface frame and a pump body, wherein the surface frame is provided with an installation position for the liquid-stopping clamp and an installation position for the infusion tube;
a pump door, which is in movable connection with the main body;
a driving module for the liquid-stopping clamp, which module is arranged at the main body and includes a movable mechanism;
the liquid-stopping clamp is configured to abut against the movable mechanism which is located at a liquid-stopping working position, so as to close the liquid-stopping clamp to clamp the infusion tube for disconnecting a path for a liquid flow inside the infusion tube, when the liquid-stopping clamp is loaded at the installation position for the liquid-stopping clamp;
the liquid-stopping clamp is further configured to release the infusion tube for connecting the path for the liquid flow inside the infusion tube, when the movable mechanism is switched from the liquid-stopping working position to a connecting working position under a driving force;
the liquid-stopping clamp is further closed to clamp the infusion tube for disconnecting the path for the liquid flow inside the infusion tube, when the movable mechanism is switched from the connecting working position to the liquid-stopping working position under the driving force.

A second aspect of an embodiment of this disclosure provides a control method for an infusion pump, which method is applied to the infusion pump, wherein the infusion pump is used in cooperation with a liquid-stopping clamp which is sleeved outside an infusion tube; the infusion pump includes: a main body, a pump door, a driving module for the liquid-stopping clamp, a sensor for sensing whether the liquid-stopping clamp is in position, a sensor for sensing a state of the pump door; wherein the main body includes a surface frame, a pump piece, and a pump body, wherein the surface frame is provided with an installation position for the liquid-stopping clamp and an installation position for the infusion tube; the pump door is in movable connection with the main body; he driving module for the liquid-stopping clamp includes a movable mechanism;
wherein the control method includes:
determining that the liquid-stopping clamp is loaded in position at the installation position for the liquid-stopping clamp, wherein the liquid-stopping clamp is configured to abut against the movable mechanism which is located at a liquid-stopping working position, when the liquid-stopping clamp is loaded in position;
determining that the pump door is in a closed state, wherein the pump door and the pump piece are configured to jointly press the infusion tube, when the pump door is in the closed state;
driving the movable mechanism to switch from the liquid-stopping working position to a connecting working position, so as to open the liquid-stopping clamp to release the infusion tube;
receiving an instruction for starting infusion, and driving the pump piece to peristaltically press the infusion tube, so as to move a liquid inside the infusion tube directionally, according to the instruction for starting infusion;
receiving an instruction for opening pump door or detecting that the pump door is opened, and driving the movable mechanism to switch from the connecting working position to the liquid-stopping working position, so as to close the liquid-stopping clamp to clamp the infusion tube;
driving a locking mechanism of the pump door to switch from a door-closing position to a door-opening position, so as to open the pump door.

A third aspect of an embodiment of this disclosure provides an infusion pump, which is used in cooperation with a liquid-stopping clamp which is sleeved outside an infusion tube; wherein the infusion pump includes:
a main body, which includes a surface frame, a pump piece, and an electric driving mechanism, wherein the surface frame is provided with an installation position for the liquid-stopping clamp and an installation position for the infusion tube;
a pump door, which is in movable connection with the main body;
a driving module for the liquid-stopping clamp, which module is arranged at the main body and includes a box body and a movable mechanism which is rotatably arranged inside the box body, wherein the installation position for the liquid-stopping clamp is connected with the box body;
wherein the liquid-stopping clamp is configured to abut against the movable mechanism which is located at a liquid-stopping working position, so as to close the liquid-stopping clamp to clamp the infusion tube for disconnecting a path for a liquid flow inside the infusion tube, when the liquid-stopping clamp is loaded at the installation position for the liquid-stopping clamp and the pump door is closed; wherein a direction for the liquid-stopping clamp abutting the movable mechanism is perpendicular to a direction for placing the infusion tube;
the liquid-stopping clamp is further configured to release the infusion tube for connecting the path for the liquid flow inside the infusion tube, when the electric driving mechanism drives the liquid-stopping clamp to rotate to a connecting working position;
the liquid-stopping clamp is further closed to clamp the infusion tube for disconnecting the path for the liquid flow inside the infusion tube, when the movable mechanism is rotated reversely from the connecting working position to a liquid-stopping working position under a driving force.

A fourth aspect of an embodiment of this disclosure provides an infusion pump, which is used in cooperation with a liquid-stopping clamp which is sleeved outside an infusion tube; wherein the infusion pump includes:
a main body, which includes a surface frame and a pump piece, wherein the surface frame is provided with an installation position for the liquid-stopping clamp and an installation position for the infusion tube;
a pump door, which is in movable connection with the main body;
a driving module for the liquid-stopping clamp, which module is arranged at the main body and includes a box body, and a movable mechanism and an elastic member, which are arranged inside the box body, wherein one end of the elastic member is connected with the movable mechanism, the other end of the elastic member is connected with the box body, wherein the installation position for the liquid-stopping clamp is connected with the box body;
the elastic member is in a first pressed state, and the liquid-stopping clamp is configured to abut against the movable mechanism which is located at a liquid-stopping working position, so as to close the liquid-stopping clamp to clamp the infusion tube for disconnecting a path for a liquid flow inside the infusion tube, when the liquid-stopping clamp is loaded at the installation position for the liquid-stopping clamp; wherein a direction for the liquid-stopping clamp abutting the movable mechanism is perpendicular to a direction for placing the infusion tube;
the pump door is configured to push the movable mechanism to move, so as to press the elastic member, when the pump door is closed; wherein the elastic member is in a second pressed state, and the liquid-stopping clamp is configured to release the infusion tube for connecting the path for the liquid flow inside the infusion tube, when the movable mechanism is moved to a connecting working position; wherein a pressed amount of the elastic member in the first pressed state is smaller than that in the second pressed state;
when the pump door is opened, the elastic member is configured to push the movable mechanism under its own elastic force, so as to switch the movable mechanism from the connecting working position to the liquid-stopping working position, such that the liquid-stopping clamp is closed to clamp the infusion tube for disconnecting the path for the liquid flow inside the infusion tube.

A fifth aspect of an embodiment of this disclosure provides an infusion pump, which is used in cooperation with an infusion tube and a liquid-stopping component capable of being disassembled; the infusion pump includes a main body and a pump door which are in movable connection with each other, one side of the main body is provided with an installation position for the infusion tube; wherein an area for pressing the infusion tube, at least one pressure detection area, and at least one bubble detection area, are arranged along the installation position for the infusion tube;
the infusion pump is configured to drive the liquid-stopping component for a shape adjustment, so as to enable a section of the infusion tube, which section is located upstream or downstream of the area for pressing the infusion tube, to switch between a connecting state and a disconnecting state, when the infusion tube is assembled with the liquid-stopping component and loaded at the installation position for the infusion tube;
the section of the infusion tube, which section is located upstream or downstream of the area for pressing the infusion tube, is in the connecting state, when the infusion tube is individually loaded at the installation position for the infusion tube.

A sixth aspect of an embodiment of this disclosure provides an infusion pump, including a main body and a pump door which are in movable connection with each other, one side of the main body is provided with an installation position for an infusion tube; wherein an area for pressing the infusion tube, at least one pressure detection area, and at least one bubble detection area, are arranged along the installation position for the infusion tube;
wherein the infusion pump further includes a split-type liquid-stopping component, which includes a liquid-stopping fixed end and a liquid-stopping movable end, which are used in cooperation with each other, wherein the liquid-stopping fixed end is arranged at the main body, and the liquid-stopping movable end is arranged at the infusion tube;
before starting infusion, the liquid-stopping movable end is correctly combined with the liquid-stopping fixed end, and the liquid-stopping movable end is maintained in a clamp state for disconnecting a path for a liquid flow inside the infusion tube;
when starting the infusion, the liquid-stopping fixed end is configured to drive the liquid-stopping movable end to switch to a release state for connecting the path for the liquid flow inside the infusion tube;
when completing the infusion, and receiving an instruction for opening pump door or detecting that the pump door is opened, the liquid-stopping fixed end is configured to drive the liquid-stopping movable end to switch to the clamp state, for disconnecting the path for the liquid flow inside the infusion tube again.

A seventh aspect of an embodiment of this disclosure provides an infusion pump, including a main body and a pump door which are in movable connection with each other, the main body is provided with an installation position for an infusion tube; wherein an area for pressing the infusion tube, at least one pressure detection area, and at least one bubble detection area, are arranged along the installation position for the infusion tube;
the infusion pump is configured to drive a liquid-stopping component capable of being disassembled to a liquid-stopping position, so as to enable a section of the infusion tube, which section is located upstream or downstream of the area for pressing the infusion tube, to be in a disconnecting state, and to enable a section of the infusion tube, which section is located inside the area for pressing the infusion tube, to be in a connecting state, when the liquid-stopping component, which carries the infusion tube, is loaded at the installation position for the infusion tube;
when the pump door is closed, the pump door and a pump piece of the main body are configured to jointly press the infusion tube, so as to enable the section of the infusion tube, which section is located inside the area for pressing the infusion tube, to be in the disconnecting state; wherein at any time point after the pump door is closed, the liquid-stopping component is switched to a connecting position, so as to enable the section of the infusion tube, which section is located upstream or downstream of the area for pressing the infusion tube, to be in the connecting state;
when the pump door is triggered to open, the liquid-stopping component is firstly switched to a liquid-stopping position, so as to enable the section of the infusion tube, which section is located upstream or downstream of the area for pressing the infusion tube, to be in the disconnecting state, and then the pump door is opened, so as to enable the section of the infusion tube, which section is located inside the area for pressing the infusion tube, to return to the connecting state.

The embodiment of this disclosure provides a control method for an infusion pump and an infusion pump, which is used in cooperation with a liquid-stopping clamp through a movable mechanism, such that in the driving aspect, the liquid disconnecting and connecting of the liquid-stopping clamp can be controlled through just controlling the movable mechanism, rather than controlling the liquid-stopping clamp and the movable mechanism separately, which simplifies the control step. Furthermore, in the liquid-stopping effect aspect, in addition to stopping the liquid before or after the infusion is started, the liquid-stopping clamp and the infusion tube are maintained tightly closed during the removal and loading of the infusion tube for stopping the liquid, which prevents the liquid inside the infusion tube from dripping out and polluting the environment. For the entire infusion pump, since the liquid-stopping clamp and the movable mechanism do not require to be arranged parallelly, but can be stacked in the direction in which the pump piece presses the infusion tube, the space in the length direction of the infusion pump can be saved, which is conducive to the miniaturization of the entire infusion pump. In summary, using the solutions of the embodiments of this disclosure can not only ensure the liquid-stopping demand of normal infusion, but also simplify the workflow of the liquid-stopping operation, which is conducive to automatic liquid stopping during tube loading and removal, and also facilitates the miniaturization design of the infusion pump.

### BRIEF DESCRIPTION OF THE DRAWINGS

FIG. 1 is a structural diagram of an infusion system provided in an embodiment of this disclosure, with an infusion pump in an open state.
FIG. 2 is a partially enlarged view of A in FIG. 1.
FIG. 3 is a structural diagram of a driving module for a liquid-stopping clamp from a perspective shown in FIG. 1.
FIG. 4 is an exploded view of FIG. 3.
FIG. 5 is a B-B sectional view of FIG. 3.
FIG. 6 is a structural diagram of the driving module for the liquid-stopping clamp shown in FIG. 3 from another perspective.
FIG. 7 is a structural diagram of a movable mechanism shown in FIG. 4.
FIG. 8 is a schematic diagram from a perspective where a liquid-stopping clamp is loaded at an installation position for the liquid-stopping clamp and a movable mechanism is in a liquid-stopping working position as shown in FIG. 1, omitting a first sub-box body.
FIG. 9 is a schematic diagram from another perspective where a liquid-stopping clamp is loaded at an installation position for the liquid-stopping clamp and a movable mechanism is in a liquid-stopping working position as shown in FIG. 1, omitting a second sub-box body.
FIG. 10 is a schematic diagram of a cooperation relationship between a liquid-stopping clamp and a driving module for the liquid-stopping clamp, when a movable mechanism is in a connecting working position.
FIG. 11 is a schematic diagram of a cooperation relationship between a driving module for a liquid-stopping clamp, an electric driving mechanism, and a guide member shown in FIG. 1, wherein a movable mechanism in the figure is in a liquid-stopping working position.
FIG. 12 is a schematic diagram of a cooperation relationship between a driving module for a liquid-stopping clamp, an electric driving mechanism, and a guide member shown in FIG. 1, wherein a movable mechanism in the figure is in a connecting working position.
FIG. 13 is a schematic diagram of a cooperation relationship between a liquid-stopping clamp and an infusion tube shown in FIG. 1.
FIG. 14 is a structural diagram of a liquid-stopping clamp shown in FIG. 13.
FIG. 15 is a partially enlarged view of position C in FIG. 14.
FIG. 16 is a flowchart of a control method for an infusion pump provided in an embodiment of this disclosure.
FIG. 17 is a structural diagram of another infusion pump provided in an embodiment of this disclosure.

### DESCRIPTION OF THE PREFERRED EMBODIMENTS

It should be noted that without conflicts, the embodiments and technical features in the embodiments in this disclosure can be combined with each other. The detailed description in the specific embodiments should be understood as an explanation of the purpose of this disclosure and should not be considered as an improper limitation of this disclosure.

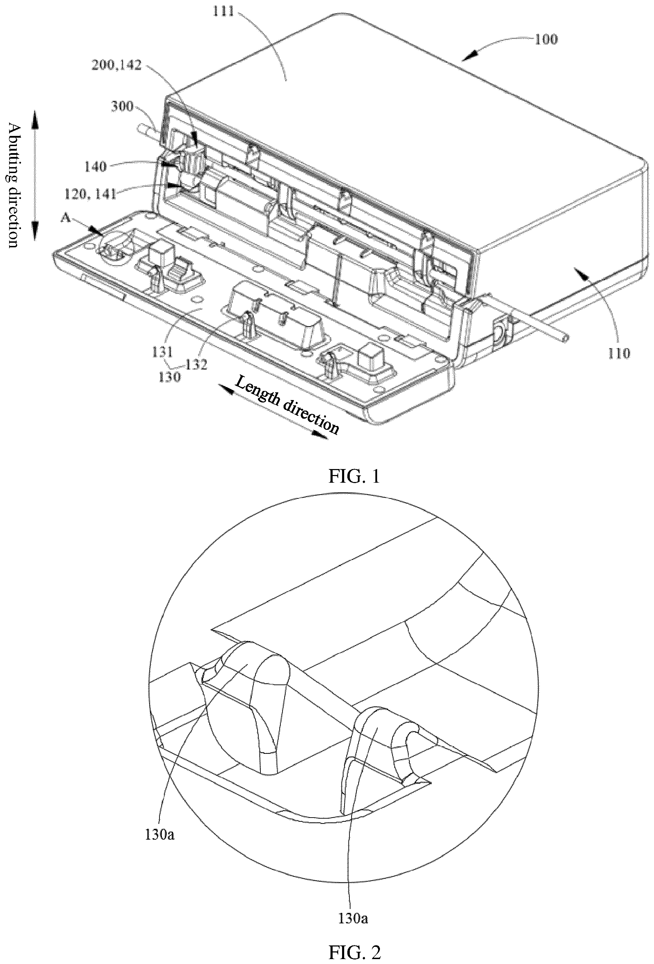

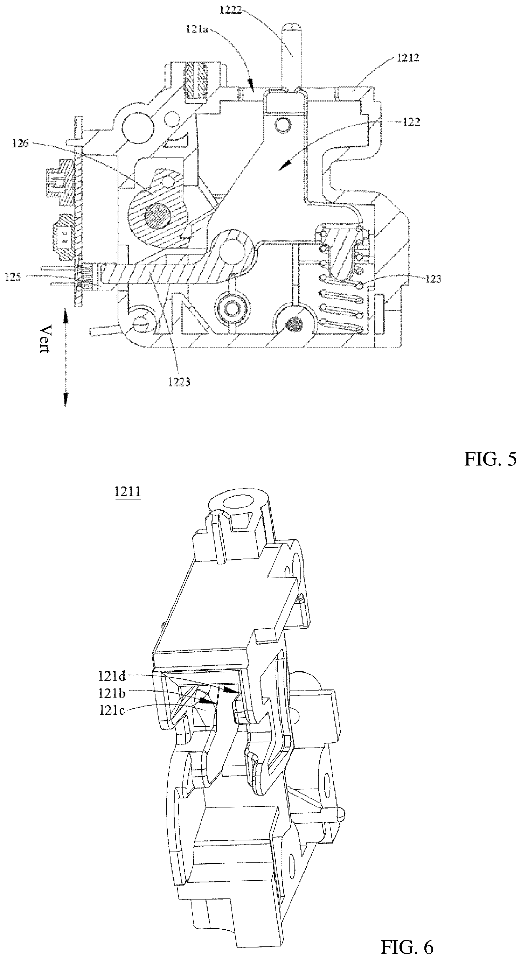

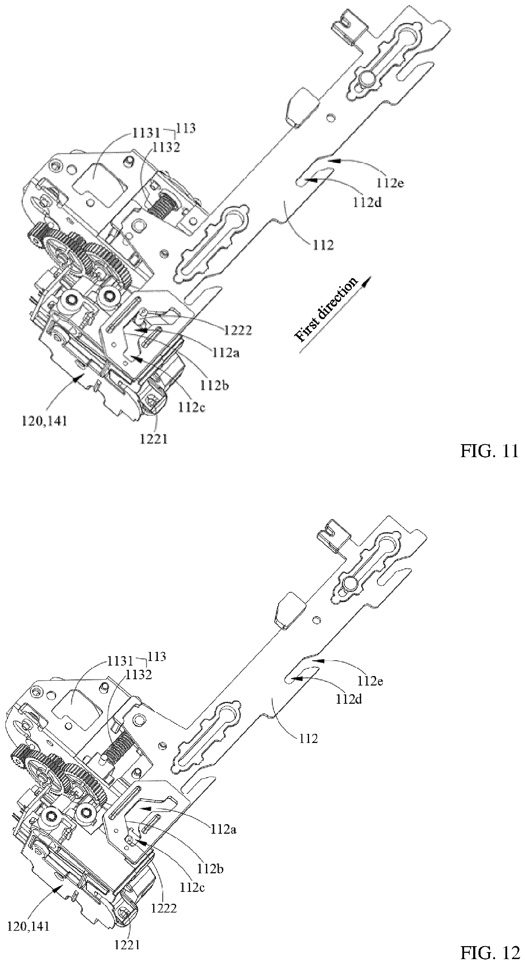

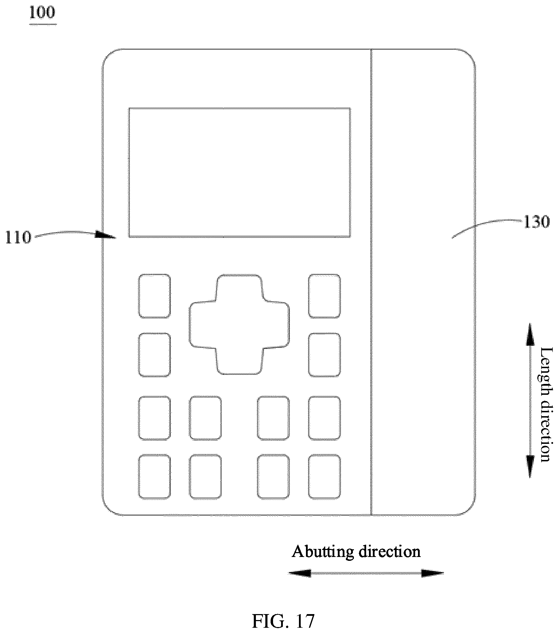

In the description of this disclosure, "length direction" and "direction for abutting" are based on directions shown in FIGS. 1 and 17, "perpendicular direction" is based on a direction shown in FIG. 5, and "first direction" is based on a direction shown in FIG. 11. It should be understood that these directional terms are only for the convenience of describing this disclosure and simplifying the description, and do not indicate or imply that the device or component referred to must have a specific orientation or be constructed and operated in a specific orientation, therefore it cannot be understood as a limitation on this disclosure.

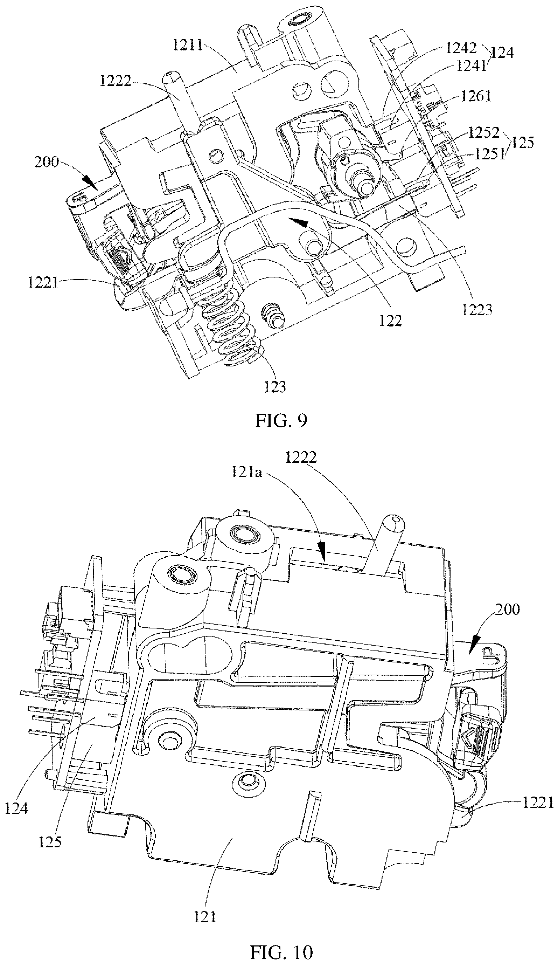

The embodiment of this disclosure provides an infusion pump 100, which is used in cooperation with a liquid-stopping clamp 200 which is sleeved outside an infusion tube 300. Referring to FIGS. 1, 3, and 10, the infusion pump 100 includes a main body 110, a pump door 130, and a driving module for the liquid-stopping clamp 120. The main body 110 includes a surface frame 111 and a pump body, wherein the surface frame 111 is provided with an installation position for the liquid-stopping clamp and an installation position for the infusion tube. The pump door 130 is in movable connection with the main body 110 to selectively avoid or block the installation position for the liquid-stopping clamp and the installation position for the infusion tube. That is to say, when the pump door 130 is opened, the pump door 130 avoids the installation position for the liquid-stopping clamp and the installation position for the infusion tube, so that the user can disassemble the liquid-stopping clamp 200 and the infusion tube 300. When the pump door 130 is closed, the pump door 130 blocks the installation position for the liquid-stopping clamp and the installation position for the infusion tube, so as to prevent the user from accidentally touching the liquid-stopping clamp 200 and infusion tube 300. The driving module for the liquid-stopping clamp 120 is arranged at the main body 110, and the driving module for the liquid-stopping clamp 120 includes a movable mechanism 122. When the liquid-stopping clamp 200 is loaded at the installation position for the liquid-stopping clamp 200, the liquid-stopping clamp 200 abuts against the movable mechanism 122 which is located at the liquid-stopping working position, so as to close the liquid-stopping clamp 200 to clamp the infusion tube 300 for disconnecting a path for a liquid flow inside the infusion tube 300. That is to say, after the liquid-stopping clamp 200 is installed at the installation position for the liquid-stopping clamp 200, under an abutting force of the movable mechanism 122, clamp arms at both ends of the liquid-stopping clamp 200 for clamping the infusion tube 300 remain in a state for clamping the infusion tube 300, so as to maintain the infusion tube 300 in a liquid-stopping state. When the movable mechanism 122 is switched from the liquid-stopping working position to a connecting working position under a driving force, the liquid-stopping clamp 200 releases the infusion tube 300 to connect the path for the liquid flow inside the infusion tube 300. That is to say, at this time, the clamp arms at both ends of the liquid-stopping clamp 200 can have a release space with a certain distance, thereby releasing the infusion tube 300 to connect the path for the liquid flow inside the infusion tube 300. At this time, medicine liquid can flow inside the infusion tube 300, the infusion pump 100 can perform normal infusion. When the movable mechanism 122 is switched from the connecting working position to the liquid-stopping working position under the driving force, the liquid-stopping clamp 200 is closed to clamp the infusion tube 300 for disconnecting the path for the liquid flow inside the infusion tube 300. That is to say, after the movable mechanism 122 is switched to the liquid-stopping working position, the liquid-stopping clamp 200 clamps the infusion tube 300 again to restore the infusion tube 300 to the liquid-stopping state.

The infusion pump 100 of the embodiment of this disclosure is mainly configured to control a parameter, such as infusion flow rate, so that the medicine liquid can be accurately and safely injected into a body of patient or diseased animal at a preset rate and dosage.

The main body 110 not only includes the surface frame 111 and the pump body, but also includes a processor, a power module, and other structures. The processor is a control center of the infusion pump 100, which can use various processor chips with signal input, output, and processing capabilities as control devices. It can also use electrical signal control methods or software control methods to control the corresponding modules, components, or mechanisms, so as to enable the infusion pump 100 to achieve corresponding functions.

"Abutting against" mainly refers to a contact between the movable mechanism 122 and the liquid-stopping clamp 200, which contact provides a support for the liquid-stopping clamp 200. When the liquid-stopping clamp 200 abuts against the movable mechanism 122 which is located at the liquid-stopping working position, the movable mechanism 122 can abut against the liquid-stopping clamp 200 without a driving force, or can abut against the liquid-stopping clamp 200 under the driving force.

Specifically, before infusion, the infusion tube 300 is loaded at the installation position for the infusion tube firstly, and then the liquid-stopping clamp 200 is loaded at the installation position for the liquid-stopping clamp. At this time, the movable mechanism 122 maintains the infusion tube 300 in the liquid-stopping state by abutting against the liquid-stopping clamp 200, which can prevent the medicine liquid from flowing freely inside the infusion tube 300. When the infusion is started, the movable mechanism 122 is switched from the liquid-stopping working position to the connecting working position under the driving force. The liquid-stopping clamp 200 releases the infusion tube 300, so as to enable the normal infusion of medicine liquid along the infusion tube 300. When the infusion is completed and the infusion tube 300 and the liquid-stopping clamp 200 need to be removed, the movable mechanism 122 is switched from the connecting working position to the liquid-stopping working position. The movable mechanism 122 abuts against the liquid-stopping clamp 200 again for disconnecting the path for the liquid flow inside the infusion tube 300, thereby preventing abnormal infusion or dripping of medicine liquid in the hospital environment. That is to say, before starting the infusion and after completing the infusion, the movable mechanism 122 is at the liquid-stopping working position. Therefore, it is not necessary to manually close the liquid-stopping clamp 200 to keep the infusion tube 300 in the liquid-stopping state. While facilitating the liquid stopping, occurrence in which the user fails to close the liquid-stopping clamp 200 in a timely manner or even forgets to close the liquid-stopping clamp 200, can be avoided. In addition, since the driving module for the liquid-stopping clamp 120 in an embodiment of this disclosure can be configured to insert the liquid-stopping clamp 200 and stop the liquid inside the infusion tube 300, there is no need to install other liquid-stopping structures at the infusion pump 100, which can save the installation space of the infusion pump 100 in the length direction and make the overall structure of the infusion pump 100 more compact.

During the switch process between the connecting working position and the liquid-stopping working position, the movable mechanism 122 can rotate or move rectilinearly, relative to the surface frame 111.

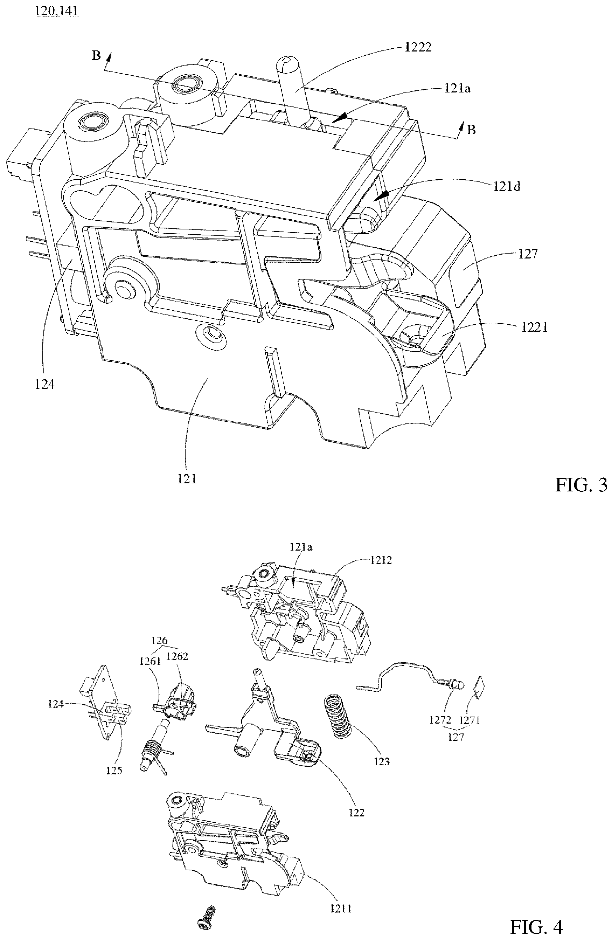

Please refer to FIGS. 3 to 5. In one embodiment, the driving module for the liquid-stopping clamp 120 includes a box body 121, and the movable mechanism 122 is movably arranged inside the box body 121. The installation position for the liquid-stopping clamp is connected with the box body 121, which is equivalent to that the installation position for the liquid-stopping clamp is arranged inside the box body 121. Arranging the box body 121 not only facilitates the installation of component, such as the movable mechanism 122, but also facilitates the installation of the driving module for the liquid-stopping clamp 120 at the main body 110.

Please refer to FIGS. 3 to 5. In an embodiment, the box body 121 includes a first sub-box body 1211 and a second sub-box body 1212. The first sub-box body 1211 and the second sub-box body 1212 are enclosed to form an inner cavity, which facilitates the installation of component, such as the movable mechanism 122. It can be understood that in some embodiments, the box body 121 can also be an integral structure or other structural form, as long as the component, such as the movable mechanism 122, can be installed in the box body 121.

In some embodiments, the driving module for the liquid-stopping clamp 120 may also have no box body 121, which means that component, such as the movable mechanism 122 can be directly installed at the main body 110.

The driving force can be either electric driving force or mechanical driving force, or a combination thereof, as long as the movable mechanism 122 can be driven to switch between the liquid-stopping working position and the connecting working position. There are various ways for switching the movable mechanism 122 from the connecting working position to the liquid-stopping working position. For example, please refer to FIGS. 5, 8, 9, and 12. In one embodiment, The driving module for the liquid-stopping clamp 120 further includes an elastic member 123 and an electric driving mechanism 113. The electric driving mechanism 113 is configured to provide an electric driving force to drive the movable mechanism 122 to switch from the liquid-stopping working position to the connecting working position. The elastic member 123 is configured to provide an elastic force to drive the movable mechanism 122 to switch from the connecting working position to the liquid-stopping working position. That is to say, the movable mechanism 122 is switched from the liquid-stopping working position to the connecting working position under the driving force of the electric driving mechanism 113, then the liquid-stopping clamp 200 releases the infusion tube 300, and the infusion tube 300 is switched from the liquid-stopping state to the connecting state. However, when the movable mechanism 122 needs to be switched from the connecting working position to the liquid-stopping working position, the movable mechanism 122 is switched from the connecting working position to the liquid-stopping working position under the elastic force of the elastic member 123, that is, under the mechanical driving force. When the movable mechanism 122 is switched from the connecting working position to the liquid-stopping working position under the elastic force of the elastic member 123, the liquid-stopping clamp 200 clamps the infusion tube 300, and the infusion tube 300 is switched from the connecting state to the liquid-stopping state.

In some embodiments, the electric driving mechanism 113 can drive the movable mechanism 122 to switch from the connecting working position to the liquid-stopping working position, while the elastic member 123 drives the movable mechanism 122 to switch from the liquid-stopping working position to the connecting working position. Optionally, the elastic member 123 can drive the movable mechanism 122 to switch from the liquid-stopping working position to the connecting working position, or drive the movable mechanism 122 to switch from the connecting working position to the liquid-stopping working position. Optionally, the electric driving mechanism 113 can drive the movable mechanism 122 to switch from the liquid-stopping working position to the connecting working position, or drive the movable mechanism 122 to switch from the connecting working position to the liquid-stopping working position.

In an embodiment, when the liquid-stopping clamp 200 is loaded at the installation position for the liquid-stopping clamp, the movable mechanism 122 is located at the liquid-stopping working position and the pump door 130 is closed; the liquid-stopping clamp 200 abuts against the movable mechanism 122 which is located at a liquid-stopping working position, so as to close the liquid-stopping clamp 200 to clamp the infusion tube 300 for disconnecting the path for the liquid flow inside the infusion tube 300. That is to say, when the pump door 130 is closed but the infusion is not started, the infusion tube 300 can remain in the liquid-stopping state. The liquid-stopping clamp 200 is mainly configured to clamp or release the infusion tube 300 by cooperating with the movable mechanism 122, and is not affected by the pump door 130.

In an embodiment, when the movable mechanism 122 is in the connecting working position and the pump door 130 is triggered to open, the movable mechanism 122 is switched from the connecting working position to the liquid-stopping working position under the driving force, so as to enable the liquid-stopping clamp 200 to close to clamp the infusion tube 300 for disconnecting the path for the liquid flow inside the infusion tube 300. In other words, as long as the pump door 130 is opened, the infusion tube 300 is switched to the liquid-stopping state, thereby further improving the liquid-stopping effect.

When the movable mechanism 122 is switched to the connecting working position, it can be separated from the liquid-stopping clamp 200, or can come into contact with the liquid-stopping clamp 200, and or can also have a certain amount of abutting with the liquid-stopping clamp 200. It should be noted that when the movable mechanism 122 at the connecting working position abuts against the liquid-stopping clamp 200, the liquid-stopping clamp 200 also applies a certain clamp force to the infusion tube 300. The infusion tube 300 may be deformed to a certain extent under the clamp force of the liquid-stopping clamp 200, but the infusion tube 300 is still in the connecting state, and the medicine liquid can flow inside the infusion tube 300.

Please refer to FIGS. 1, 2, 13, and 14. In an embodiment, the liquid-stopping clamp 200 includes a first clamp arm 210, a second clamp arm 220, a first clamp member 240, and a second clamp member 250. The first clamp arm 210 and the second clamp arm 220 are connected with each other, the first clamp member 240 is arranged at the first clamp arm 210, and the second clamp member 250 is arranged at the second clamp arm 220. The pump door 130 includes a pushing member 130a. When the liquid-stopping clamp 200 is loaded at the installation position for the liquid-stopping clamp, the liquid-stopping clamp 200 abuts against the movable mechanism 122 which is located at the liquid-stopping working position, the first clamp member 240 is in clamp connection with the second clamp member 250, and the infusion tube 300 is maintained in the liquid-stopping state by abutting against the liquid-stopping clamp 200. When the pump door 130 is closed, the pushing member 130a pushes at least one of the first clamp member 240 or the second clamp member 250, to separate the first clamp member 240 and the second clamp member 250. After the first clamp member 240 is separated from the second clamp member 250, the liquid-stopping clamp 200 is in a semi-closed state. However, due to that the movable mechanism 122 is still at the liquid-stopping working position, it restricts the movement of the liquid-stopping clamp 200 in a release direction of the first clamp arm 210 and the second clamp arm 220, such that the movable mechanism 122 can continue to maintain the infusion tube 300 in the liquid-stopping state by abutting the liquid-stopping clamp 200. When the first clamp member 240 and the second clamp member 250 are separated and the movable mechanism 122 is switched from the liquid-stopping working position to the connecting working position under the driving force, the first clamp arm 210 and the second clamp arm 220 have a certain amount of moving space in the release direction. The liquid-stopping clamp 200 releases the infusion tube 300 to connect the path for the liquid flow inside the infusion tube 300.

Please refer to FIGS. 1, 2, 13, and 14. In an embodiment, when the movable mechanism 122 is switched from the connecting working position to the liquid-stopping working position under the driving force, the liquid-stopping clamp 200 is closed to clamp the infusion tube 300 for disconnecting the path for the liquid flow inside the infusion tube 300. When the pump door 130 is opened, the pushing member 130a is separated from the liquid-stopping clamp 200, and the first and second clamp members 240 and 250 are reconnected via clamping under their own elastic forces. That is to say, when the infusion is completed and the user opens the pump door 130, the pushing member 130a is separated from the liquid-stopping clamp 200, and the second clamp member 250 is returned to its initial position and is once again in clamp connection with the first clamp member 240. Therefore, after the liquid-stopping clamp 200 is pulled out of the box body 121, the infusion tube 300 can continue to remain in the liquid-stopping state to prevent the liquid from flowing out when the tube is pulled out. In addition, after the pump door 130 is opened, the first clamp member 240 and the second clamp member 250 can be automatically in the clamp connection to maintain the first clamp arm 210 and the second clamp arm 220 in the state of clamping the infusion tube 300, without requiring the user to manually restore the clamp connection between the first clamp member 240 and the second clamp member 250, thereby reducing user operation. At the same time, it is also convenient for the user to pull out the liquid-stopping clamp 200 from the box body 121.

Please refer to FIGS. 1, 2, 14, and 15. In a specific embodiment, the first clamp member 240 has a first abutting part 240a, and the second clamp member 250 has a second abutting part 250a. When the pump door 130 is in an open state and the liquid-stopping clamp 200 is loaded at the installation position for the liquid-stopping clamp, the first clamp member 240 and the second clamp member 250 are located at a side of the box body 121, which side is adjacent to the pump door 130. The first abutting part 240a and the second abutting part 250a abut against each other in the release direction of the liquid-stopping clamp 200. When pump door 130 is closed, the pushing member 130a pushes the second clamp member 250, and the second abutting part 250a is moved towards an elastic arm 230. In this way, the second abutting part 250a is separated from the first abutting part 240a, such that the first clamp member 240 is separated from the second clamp member 250. In some embodiments, a corresponding triggering structure can also be arranged at the driving module for the liquid-stopping clamp 120 to control the separation of the first clamp member 240 and the second clamp member 250.

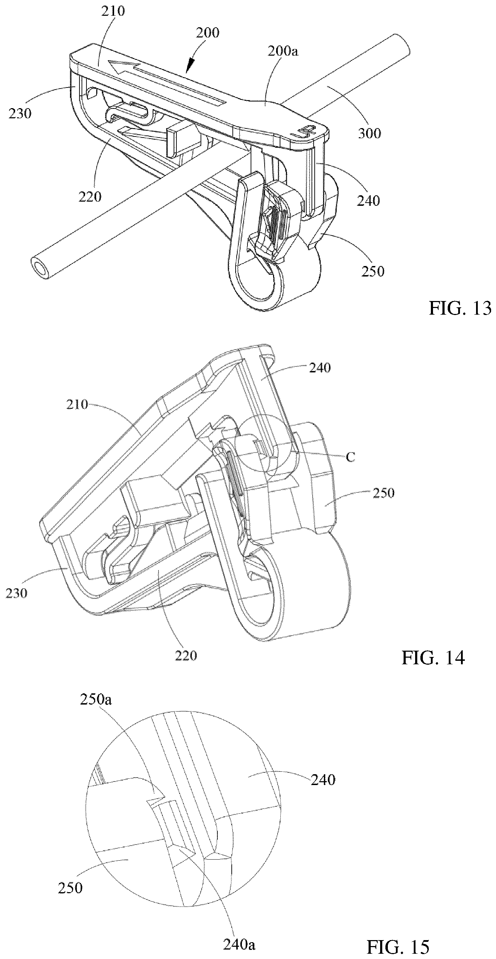

Please refer to FIGS. 14 and 15. In one embodiment, the liquid-stopping clamp 200 is an elastic liquid-stopping clamp, which has the elastic arm 230. The first clamp member 240 is arranged at an end of the first clamp arm 210, which end is away from the elastic arm 230, and the second clamp member 250 is arranged at an end of the second clamp arm 220, which end is away from the elastic arm 230. When the movable mechanism 122 is switched from the liquid-stopping working position to the connecting working position under the driving force, the elastic liquid-stopping clamp 200 releases the infusion tube 300 under its own elastic force, so as to connect the path for the liquid flow inside the infusion tube 300.

The elastic liquid-stopping clamp in FIG. 14 has two second clamp members 250 and a first clamp member 240. The two second clamp members 250 are arranged opposite to each other on both sides of the first clamp member 240, and each second clamp member 250 has one second abutting part 250a. Correspondingly, two first abutting parts 240a are arranged at the first clamp member 240, and each first abutting part 240a is matched with the second abutting part 250a of the corresponding second clamp member 250, which can improve the clamping effect of the first clamp member 240 and the second clamp member 250. It can be understood that in some embodiments, only one second clamp member 250 can be arranged.

In another embodiment, the liquid-stopping clamp 200 can also be a non-elastic liquid-stopping clamp, which means that the non-elastic liquid-stopping clamp does not have the elastic arm 230. For example, the first clamp arm 210 and the second clamp arm 220 of the non-elastic liquid-stopping clamp can be rotatably connected. When the movable mechanism 122 is switched from the liquid-stopping working position to the connecting working position under the driving force, at least one of the first clamp arm 210 and the second clamp arm 220 rotates relatively under the driving force of the movable mechanism 122, thus releasing the infusion tube 300.

Please refer to FIG. 6. In one embodiment, a side of the box body 121, which is adjacent to the pump door 130, has an insertion interface 121b that can be used for inserting into and pulling out from the liquid-stopping clamp 200. The insertion interface 121b in FIG. 6 is formed at the first sub-box body 1211, that is to say, the installation position for the liquid-stopping clamp is arranged inside the first sub-box body 12111, and the movable mechanism 122 is arranged inside the second sub-box body 1212. In other embodiments, the insertion interface 121b can also be formed at the second sub-box body 1212. A guide slop 121c for guiding the liquid-stopping clamp 200 into the insertion interface 121b is arranged at the insertion interface 121b. The guide slope 121c can be an inclined plane or a smooth inclined surface. When inserting the liquid-stopping clamp 200 from the insertion interface 121b into the box body 121, the liquid-stopping clamp 200 can slide along the guide slope 121c into the box body 121, allowing the user to insert the liquid-stopping clamp 200 into the box body 121 without deliberately aligning it. Furthermore, it is beneficial to reduce the difficulty of inserting the liquid-stopping clamp 200 into the box body 121, and to achieve blind insertion of the liquid-stopping clamp 200, and to reduce misoperation.

Please refer to FIGS. 6 and 13. In one embodiment, a second position-limiting groove 121d is arranged at a side wall of the box body 121, and the liquid-stopping clamp 200 has a position-limiting part 200a. When the liquid-stopping clamp 200 is inserted into the box body 121 in position, the liquid-stopping clamp 200 is in clamp connection with the position-limiting groove 121d through the position-limiting part 200a. Therefore, the liquid-stopping clamp 200 can play a position-limiting role, thereby achieving accurate and reliable positioning of the liquid-stopping clamp 200 and reducing the oscillation of the liquid-stopping clamp 200.

Please refer to FIG. 6. In an embodiment, the guide slope 121c and the position-limiting groove 121d are located on opposite sides of the insertion interface 121b, respectively. This not only improves the compactness of the box body 121, but also prevents interference between the guide slope 121c and the position-limiting groove 121d.

In an embodiment, a color of the surface frame 111 is different from a color of the liquid-stopping clamp 200, and a color of at least a portion of the box body 121, which portion is at the insertion interface 121b, is the same as the color of the liquid-stopping clamp 200. Therefore, it can facilitate the user to quickly identify the insertion position of the liquid-stopping clamp 200, thereby improving the installation efficiency of the liquid-stopping clamp 200. For example, since the insertion interface 121b of the driving module for the liquid-stopping clamp 120 in FIG. 3 is arranged at the first sub-box body 1211, for facilitating arrangement, the color of the entire first sub-box body 1211 can be the same as the liquid-stopping clamp 200. In other embodiments, the color of the entire box body 121 can also be same as the liquid-stopping clamp 200.

Please refer to FIGS. 3, 5, 8, and 12. In one embodiment, the electric driving mechanism 113 includes a driving motor 1131 and a transmission member 1132 which is in drive connection with the driving motor 1131, the main body 110 further includes a guide member 112, which is connected with the transmission member 1132 and located outside the box body 121. A wall of the box body 121 has a first direction-guiding groove 121a that connects inside and outside of the box body 121. The first direction-guiding groove 121a in FIG. 3 is arranged at a top wall of the box body 121. In some embodiments, the first direction-guiding groove 121a can also be arranged at the side wall or bottom wall of the box body 121. In this embodiment, the first direction-guiding groove 121a is formed by the joint enclosure of the first sub-box body 1211 and the second sub-box body 1212. In other embodiments, the first direction-guiding groove 121a can also be separately formed at the first sub-box body 1211 or the second sub-box body 1212. The movable mechanism 122 includes an abutting member 1221 and a direction-guiding member 1222 which is connected with the abutting member 1221. The direction-guiding member 1222 passes through the first direction-guiding groove 121a and extends out of the box body 121. When the movable mechanism 122 is at the liquid-stopping working position, the abutting member 1221 abuts against the liquid-stopping clamp 200, and the direction-guiding member 1222 is at a first initial position in the first direction-guiding groove 121a. The driving motor 1131 drives the transmission member 1132 to drive the guide member 112 to move rectilinearly in a first direction. The guide member 112 guides the direction-guiding member 1222 to move from the first initial position along the extension direction of the first direction-guiding groove 121a to a first target position in the first direction-guiding groove 121a, then the movable mechanism 122 is switched from the liquid-stopping working position to the connecting working position. That is to say, the electric driving mechanism 113 can drive the movable mechanism 122 to move through the guide member 112 (which is used as an intermediate member), which facilitates guiding the direction-guiding member 1222 to move from the first initial position in the first direction-guiding groove 121a to the first target position in the first direction-guiding groove 121a.

The transmission member 1132 can be a ball screw, a gear and a rack, or a combination of various parts, such as a combination of ball screws and multiple gears, as long as it can drive the direction-guiding member 1222 to move rectilinearly, under the drive of the driving motor 1131.

There are various ways in which, the guide member 112 guides the direction-guiding member 1222 from the first initial position to the first target position, for example, refer to FIGS. 11 and 12. In one embodiment, the guide member 112 pushes the direction-guiding member 1222 from the first initial position to the first target position. In other words, during the process in which the guide member 112 moves rectilinearly towards the first direction, the guide member 112 abuts against the direction-guiding member 1222 and applies a pushing force to the direction-guiding member 1222, so as to push the direction-guiding member 1222 from the first initial position to the first target position.

In another embodiment, the driving module for the liquid-stopping clamp 120 further includes an elastic member installed inside the box body 121 and connected with the movable mechanism 122. When the movable mechanism 122 is in a structural form that rotates relative to the box body 121, the elastic member can be a torsion spring. When the movable mechanism 122 is in a structural form that moves rectilinearly relative to the box body 121, the elastic member can be a compression spring, a tension spring, etc. When the movable mechanism 122 is located at the liquid-stopping working position, the guide member 112 blocks the direction-guiding member 1222 to generate an elastic deformation. That is to say, at this time, the guide member 112 blocks a portion of the first direction-guiding groove 121a to prevent the direction-guiding member 1222 from moving towards the first target position. When the driving motor 1131 drives the transmission member 1132 to move the guide member 112 rectilinearly towards the first direction, the guide member 112 avoids the direction-guiding member 1222, that is, the guide member 112 is moved away from the position blocking the first direction-guiding groove 121a, so that the direction-guiding member 1222 can move to the first target position under the elastic force of the elastic member.

Please refer to FIGS. 11 and 12. In one embodiment, the guide member 112 has a direction-guiding side 112b, and the guide member 112 guides the direction-guiding member 1222 to move from the first initial position to the first target position through the direction-guiding side 112b. The direction of linear movement of the guide member 112 is perpendicular to the extension direction of the direction-guiding groove, and the extension direction of the direction-guiding side 112b is obliquely intersected with the direction of linear movement of the guide member 112 and the extension direction of the first direction-guiding groove 121a, respectively.

Specifically, when the guide member 112 moves rectilinearly towards the first direction, the direction-guiding side 112b passes through the first direction-guiding groove 121a from a side of the first direction-guiding groove 121a, which side is along the extension direction. As the extension direction of the direction-guiding side 112b is obliquely intersected with the extension direction of the first direction-guiding groove 121a, during the process of the direction-guiding side 112b passing through the first direction-guiding groove 121a, a position, where the direction-guiding side 112b overlaps with the first direction-guiding groove 121a, moves along the extension direction of the first direction-guiding groove 121a, so that the direction-guiding side 112b can guide the direction-guiding member 1222 from the first initial position to the first target position.

The advantage of arranging the direction of linear movement of the guide member 112 to be perpendicular to the extension direction of the direction-guiding groove is that the guide member 112 can be arranged to move rectilinearly along the length direction of the infusion pump 100. Therefore, the space in the length direction of the infusion pump 100 can be fully utilized to facilitate the arrangement of the electric driving mechanism 113, thereby making the overall structure of the infusion pump 100 more compact.

In some embodiments, the direction of linear movement of the guide member 112 can also be parallel or obliquely intersected with the extension direction of the direction-guiding groove, as long as it is ensured that the guide member 112 can guide the direction-guiding member 1222 from the first initial position to the first target position.

In addition, it should be noted that there is a certain error allowed for both perpendicular and parallel arrangement in this disclosure, which means that perpendicular arrangement includes approximately perpendicular arrangement and the parallel arrangement includes approximately parallel arrangement.

Please refer to FIGS. 11 and 12. In one embodiment, the guide member 112 has a first position-limiting groove 112a, the direction-guiding member 1222 penetrates into and is arranged inside the first position-limiting groove 112a, and a corresponding part of the side wall of the first position-limiting groove 112a is the direction-guiding side 112b. The first position-limiting groove 112a can play a position-limiting role on the guide member 112 by cooperating with the direction-guiding member 1222, so as to prevent significant deviation in the position of the guide member 112.

Please refer to FIGS. 11 and 12. In one embodiment, the guide member 112 also has a first locking groove 112c which is connected with the first position-limiting groove 112a. When the movable mechanism 122 is located at the connecting working position, the direction-guiding member 1222 is located inside the first locking groove 112c and in locking corporation with the first locking groove 112c. Arranging the first locking groove 112c can facilitate the locking of the movable mechanism 122 at the connecting working position.

Please refer to FIGS. 5, 8, 11, and 12. In one embodiment, the movable mechanism 122 can be rotatably arranged inside the box body 121. When the liquid-stopping clamp 200 is loaded at the installation position for the liquid-stopping clamp 200 and the pump door 130 is closed, the liquid-stopping clamp 200 and the movable mechanism 122 which is located at the liquid-stopping working position abuts against each other in the perpendicular direction. That is to say, the entire movable mechanism 122 can be located above or below the liquid-stopping clamp 200, the movable mechanism 122 abuts against the liquid-stopping clamp 200 from above or below, or a portion of the movable mechanism 122 is located above or below the liquid-stopping clamp 200, and the portion of the movable mechanism 122 which is located above or below the liquid-stopping clamp 200, abuts against the liquid-stopping clamp 200. When the electric driving mechanism 113 drives the liquid-stopping clamp 200 to rotate to the connecting working position, the liquid-stopping clamp 200 releases the infusion tube 300. That is to say, the movable mechanism 122 is rotated by the electric driving mechanism 113 to switch from the liquid-stopping work position to the connecting working position. When the movable mechanism 122 is driven by the driving force to switch from the connecting working position to the liquid-stopping work position through reverse rotation, the liquid-stopping clamp 200 is closed to clamp the infusion tube 300.

There are various types of infusion pumps 100, but a direction for the liquid-stopping clamp 200 abutting the movable mechanism 122, which is at the installation position for the liquid-stopping clamp, is perpendicular to a direction for placing the infusion tube. For example, referring to FIG. 1, for the infusion pump 100 in the above embodiment, when the direction for the liquid-stopping clamp 200 abutting the movable mechanism 122, is perpendicular to the direction for placing the infusion tube, the liquid-stopping clamp 200 actually abuts against the movable mechanism 122, which is located at the liquid-stopping working position, in the perpendicular direction (as shown in FIG. 5). Please refer to FIG. 17. There is also a type of infusion pump 100 that is a vertical pump (which can be understood as the infusion pump in the above embodiment rotating 90 ° counterclockwise). When the direction for the liquid-stopping clamp 200 of the vertical pump abutting the movable mechanism 122 is perpendicular to the direction for placing the infusion tube, the liquid-stopping clamp 200 actually abuts against the movable mechanism 122 which is located at the liquid-stopping working position, in the horizontal direction (as shown in FIG. 5). Usually, the direction for placing the infusion tube is consistent with the length direction of the infusion pump.

The driving force for the reverse rotation of the movable mechanism 122 can be provided by the electric driving mechanism 113, or by a reset member, such as an elastic member. For example, please refer to FIGS. 5, 8, 11, and 12. In a specific embodiment, the box body 121 is provided with an elastic member 123, one end of which is connected with the movable mechanism 122, the other end of which is connected with the box body 121, and the guide member 112 is located outside the box body 121. When the liquid-stopping clamp 200 is loaded at the installation position for the liquid-stopping clamp and the pump door 130 is closed, the elastic member 123 is in a first pressed state. The electric driving mechanism 113 drives the guide member 112 to move rectilinearly in the first direction. During the movement of the guide member 112 towards the first direction, the movable mechanism 122 is driven by the guide member 112 to rotate in a first clock-moving direction until it reaches the connecting working position. During the rotation of the movable mechanism 122 in the first clock-moving direction, the elastic member 123 is pressed until it is in a second pressed state. The electric driving mechanism 113 drives the guide member 112 to move rectilinearly towards the second direction. During the movement of the guide member 112 towards the second direction, the movable mechanism 122 is driven by the elastic member 123 to rotate in a second clock-moving direction which is opposite to the first clock-moving direction until it reaches the liquid-stopping working position. That is to say, the elastic member 123 can cooperate with the abutting member 1221 to abut against the liquid-stopping clamp 200 tightly, and can also push the movable mechanism 122 back from the connecting working position to the liquid-stopping working position. This not only improves the liquid-stopping effect, but also facilitates the resetting of the movable mechanism 122. In this embodiment, the elastic member 123 is a compression spring, and in other embodiments, the elastic member 123 can also be a torsion spring.

In the above embodiment, the first direction-guiding groove 121a is arranged on a top wall of the box body 121, the first initial position is located at one end of the first direction-guiding groove 121a, which end is away from the pump door 130, and the first target position is located at one end of the first direction-guiding groove 121a, which end is adjacent to the pump door 130.

In another specific embodiment, the first direction-guiding groove is similar arranged on the top wall of the box body, the first initial position is located at one end of the first direction-guiding groove, which end is adjacent to the pump door, the first target position is located at one end of the first direction-guiding groove, which end is away from the pump door, the abutting member faces the insertion interface, and the elastic member is located at a side of the abutting member, which side is away from the insertion interface. When the guide member is in the first initial position, the elastic member is in the first pressed state. When the driving motor drives the transmission member to move the guide member rectilinearly towards the first direction, the guide member pushes the direction-guiding member to move from the first initial position to the first target position, and the movable mechanism moves rectilinearly away from the insertion interface. When the guide member moves to the first target position, the elastic member is switched to the second pressed state. When the driving motor drives the transmission member to move the guide member rectilinearly towards the second direction, the elastic member, under its own elastic force, pushes the movable mechanism to move rectilinearly, towards a direction close to the insertion interface, so as to enable the direction-guiding member to move from the first target position to the first initial position. That is to say, the movable mechanism can also cooperate with the elastic member through a rectilinear movement.

It can be understood that in some embodiments, the elastic member 123 may not be arranged. For example, when the driving motor 1131 drives the transmission member 1132 to move the guide member 112 rectilinearly towards the second direction, the direction-guiding member 1222 can also be pulled back from the first target position to the first initial position through the guide member 112.

Please refer to FIGS. 1, 11, and 12. In one embodiment, the guide member 112 moves rectilinearly along the length direction of the infusion pump 100. The pump door 130 includes a door body 131 which is in rotational connection with the main body 110 and a locking member 132 which is arranged at the door body 131. The guide member 112 also has a second locking groove 112d which extends along the length direction of the infusion pump 100, with an opening 112e on a side which is adjacent to the pump door 130. When the pump door 130 is closed, the locking member 132 is located inside the second locking groove 112d and corresponds to the opening 112e. At this time, the movable mechanism 122 is at the liquid-stopping working position, that is to say, the locking member 132 is located at the opening 112e, and the locking member 132 can move from the opening 112e to the outside of the second locking groove 112d to open the pump door 130. When the driving motor 1131 drives the transmission member 1132 to move the guide member 112 rectilinearly in the first direction, the opening 112e is not aligned with the locking member 132, and the second locking groove 112d is in clocking cooperation with the locking member 132, the pump door 130 is locked. That is to say, during the rectilinear movement of the guide member 112 in the first direction, the pump door 130 is in a locked state and cannot be opened.

In addition, due to the fact that, during the process that the driving motor 1131 drives the transmission member 1132 to move the guide member 112 rectilinearly in the first direction, the movable mechanism 122 is also in the process of switching from the liquid-stopping working position to the connecting working position. Therefore, when the guide member 112 is used in cooperation with the pump door 130, the pump door 130 is kept in a locked state during the period from starting the infusion pump 100 to stopping the infusion pump 100, such that the safety of the infusion process can be improved.

The advantage of using the same guide member 112 in combination with both the movable mechanism 122 and the pump door 130 is that only one electric driving mechanisms 113 can be configured to control the movable mechanism 122 and pump door 130. This can reduce the number of electric driving mechanisms 113 used, save production costs, and also make the overall structure of the infusion pump 100 as compact as possible.

It can be understood that the guide member 112 may not be installed inside the main body 110. For example, in one embodiment, a rotary shaft, which is in rotational connection with the box body 121 can be installed inside the driving module for the liquid-stopping clamp 120. Some structure of the rotary shaft is located inside the box body 121 and connected with the movable mechanism 122, and one end of the rotary shaft is located outside the box body 121 and connected with the electric driving mechanism 113. It should be noted that the electric driving mechanism 113 can only have a driving motor 1131, as well as can have a driving motor 1131 and a transmission member 1132, which can be of various types. For example, the transmission member 1132 can be a combination of gears, or a combination of turbines and worms, as long as the transmission member 1132 can drive the rotary shaft to rotate. When the electric driving mechanism 113 only has a driving motor 1131, the rotary shaft is connected with the output end of the driving motor 1131, When the electric driving mechanism 113 is also provided with a transmission member 1132, the rotary shaft is connected with the transmission member 1132. That is to say, the rotary shaft is directly connected with the electric driving mechanism 113, which drives the rotary shaft to rotate, so as to enable the rotary shaft to drive the movable mechanism 122 to switch between the liquid-stopping working position and the connecting working position.

In another embodiment, the electric driving mechanism 113 includes a driving motor 1131 and a transmission member 1132 which is in drive connection with the driving motor 1131. The driving module for the liquid-stopping clamp 120 further includes a push-pull shaft. One end of the push-pull shaft is located inside the box body 121 and connected with the movable mechanism 122, and the other end of the push-pull shaft is located outside the box body 121 and connected with the transmission member 1132. The driving motor 1131 drives the transmission member 1132 to move the push-pull shaft rectilinearly, so as to switch the movable mechanism 122 between the liquid-stopping working position and the connecting working position, through the push-pull shaft. That is to say, the transmission member 1132, that can perform rectilinear movement, can be configured to move the push-pull shaft rectilinearly, so as to enable the movable mechanism 122 to switch between the liquid-stopping working position and the connecting working position through the rectilinear movement.

In an embodiment, when the pump door 130 is in an open state and the liquid-stopping clamp 200 is loaded at the installation position for the liquid-stopping clamp, the movable mechanism 122 is at the liquid-stopping working position. When the pump door 130 is closed, the pump door 130 pushes the movable mechanism 122 from the liquid-stopping working position to the connecting working position. That is to say, the pump door 130 can be configured to apply a mechanical driving force to the movable mechanism 122, so as to enable the movable mechanism 122 to switch from the liquid-stopping working position to the connecting working position.

In an embodiment, the driving module for the liquid-stopping clamp 120 further includes an elastic member which is arranged inside the box body 121, one end of the elastic member is connected with the box body 121, and the other end of the elastic member is connected with the movable mechanism 122. The movable mechanism 122 can rotate relative to the box body 121 or move rectilinearly relative to the box body 121. Correspondingly, when the movable mechanism 122 is in a structural form that rotates relative to the box body 121, the elastic member can be a torsion spring. When the movable mechanism 122 is in a structural form that moves rectilinearly relative to the box body 121, the elastic member can be a compression spring. When the liquid-stopping clamp 200 is loaded at the installation position for the liquid-stopping clamp, the elastic member is in the first pressed state, and the liquid-stopping clamp 200 abuts against the movable mechanism 122 which is located at the liquid-stopping working position in the perpendicularly direction, so as to enable the liquid-stopping clamp 200 to close to clamp the infusion tube 300 for disconnecting the path for the liquid flow inside the infusion tube 300. When pump door 130 is closed, it pushes the movable mechanism 122 to move, and the movable mechanism 122 presses the elastic member. When the movable mechanism 122 moves to the connecting working position, the elastic member is in the second pressed state, and the liquid-stopping clamp 200 releases the infusion tube 300 to connect the path for the liquid flow inside the infusion tube 300. Among them, the pressed amount of the elastic member in the first pressed state is less than the pressed amount of the elastic member in the second pressed state. When the pump door 130 is opened, the elastic member pushes the movable mechanism 122 under its own elastic force, so as to switch the movable mechanism 122 from the connecting working position to the liquid-stopping working position. The liquid-stopping clamp 200 is closed to clamp the infusion tube 300 for disconnecting the path for the liquid flow inside the infusion tube 300. That is to say, the infusion pump 100 can rely on the mechanical driving force of the pump door 130 and the elastic member to switch the movable mechanism 122 between the connecting working position and the liquid-stopping working position.

In an embodiment, the infusion pump 100 can also be provided with a switch mechanism, which is in movable connection with the main body 110. Before infusion, when the pump door 130 is in an open state, the switch mechanism and the movable mechanism 122 can cooperate through abutting, clamping, plugging, and other methods to maintain the movable mechanism 122 at the liquid-stopping working position. After the pump door 130 is closed, the user moves the switch mechanism, so as to enable the switch mechanism to cooperate with the pump door 130 for locking the pump door 130. At the same time, the switch mechanism is separated from the movable mechanism 122 to enable the movable mechanism 122 to switch to the connecting working position. After the infusion is completed, the user can move the switch mechanism to separate the switch mechanism from the pump door 130 to unlock the pump door 130. At the same time, the switch mechanism again cooperates with the movable mechanism 122 to return the movable mechanism 122 to the liquid-stopping working position, that is to say, by arranging the switch mechanism, the movable mechanism 122 can be switched to the connecting working position when pump door 130 is locked, and to the liquid-stopping working position when pump door 130 is unlocked.

Please refer to FIGS. 4, 5, 8 to 10. In one embodiment, the driving module for the liquid-stopping clamp 120 further includes a first detection member 124. When the liquid-stopping clamp 200 is inserted into the box body 121 in position, the liquid-stopping clamp 200 triggers the first detection member 124 to output first in-position information. By arranging the first detection member 124, it can be determined whether the liquid-stopping clamp 200 is properly inserted.

There are various ways in which the liquid-stopping clamp 200 triggers the first detection member 124 to output the first in-position information. For example, please refer to FIGS. 4, 5, 8 to 9. In one embodiment, the driving module for the liquid-stopping clamp 120 includes a first triggering member 126 which is movably arranged inside the box body 121. When the liquid-stopping clamp 200 is loaded at the installation position for the liquid-stopping clamp, the liquid-stopping clamp 200 pushes the first triggering member 126, so as to enable the first triggering member 126 to trigger the detection member 124 for outputting the first in-position information. That is to say, the liquid-stopping clamp 200 can trigger the first detection member 124 to output the first in-position information through the first triggering member 126 which is arranged inside the box body 121.

Please refer to FIGS. 4, 5, 8 to 9. In a specific embodiment, the first detection member 124 is a first optocoupler, which includes a first transmitting terminal 1241 and a first receiving terminal 1242 which are spaced oppositely. The first triggering member 126 is in rotational connection with the box body 121. The first triggering member 126 includes a first triggering part 1261 and an abutting top 1262 which is connected with the first triggering part 1261. When the first triggering member 126 is in an initial state, the first triggering part 1261 is located at an interval between the first transmitting terminal 1241 and the first receiving terminal 1242. At this time, the first triggering part 1261 disconnects a signal transmission path between the first transmitting terminal 1241 and the first receiving terminal 1242. The first receiving terminal 1242 is unable to receive a signal transmitted by the first transmitting terminal 1241. When the liquid-stopping clamp 200 is inserted into the box body 121 in position, the liquid-stopping clamp 200 applies a pushing force to the abutting top 1262, so as to enable the first triggering member 126 to rotate to drive the first triggering part 1261 to move out of the interval between the first transmitting terminal 1241 and the first receiving terminal 1242. After the first triggering member 126 moves out of the interval between the first transmitting terminal 1241 and the first receiving terminal 1242, the signal transmission path between the first transmitting terminal 1241 and the first receiving terminal 1242 is conducted, the first receiving terminal 1242 receives the signal transmitted by the first transmitting terminal 1241, thereby enabling the first optocoupler to output the first in-position information.

In some embodiments, it is also possible that when the first triggering member 126 is in the initial state, the first triggering part 1261 is located outside the first optocoupler, and when the liquid-stopping clamp 200 is inserted into the box body 121 in position, the liquid-stopping clamp 200 applies a pushing force to the abutting top 1262, so as to enable the first triggering member 126 to rotate to drive the first triggering part 1261 to move to the interval between the first transmitting terminal 1241 and the first receiving terminal 1242.

It can be understood that the first detection member 124 is not limited to the first optocoupler. In some embodiments, the first detection member 124 can be a Hall sensor, button switch, optical proximity sensor, pressure sensor, or ultrasonic sensor, etc.

In some embodiments, the first triggering member 126 can also trigger the first detection member 124 to output the first in-position information through a rectilinear movement.

In some embodiments, the first triggering member 126 may not be arranged, for example, a second triggering part may be arranged at the liquid-stopping clamp 200. When the liquid-stopping clamp 200 is inserted in position into the box body 121, the second triggering part extends into the interval between the first transmitting terminal 1241 and the first receiving terminal 1242 of the first optocoupler to trigger the first optocoupler to output the first in-position information. Optionally, when the liquid-stopping clamp 200 is inserted in position into the box body 121, the liquid-stopping clamp 200 is in contact with a key switch to conduct the key switch.

In an embodiment, the processor is in signal connection with the electric driving mechanism 113, which is configured to receive the first in-position information and control the electric driving mechanism 113 to drive the movable mechanism 122 to switch from the liquid-stopping working position to the connecting working position. In other words, the processor can start the infusion pump 100 after confirming that the liquid-stopping clamp 200 has been inserted in position, thereby improving the safety of the use of the infusion pump 100.

Refer to FIG. 4 and FIG. 5. In one embodiment, a light module 127 can also be arranged at the driving module for the liquid-stopping clamp 120, and the light module 127 is configured to send out an indication light or change a color of the indication light according to the first in-position information.

Specifically, the first detection member 124 can directly output the first in-position information to the light module 127, and the light module 127 sends out the indication light or change the color of the indication light after receiving the first in-position information, or the processor receives the first in-position information and control the light module 127 to send out the indication light or change the color of the indication light, so that the user can intuitively determine that the liquid-stopping clamp 200 is installed in position.

Refer to FIG. 4 and FIG. 5. In one embodiment, the light module 127 includes a warning sheet 1271 and a state indication light 1272. The box body 121 is provided with a light hole (not shown in the figure), the state indication light 1272 penetrates into and is arranged inside the light hole, and the warning sheet 1271 is shielded outside the state indication light 1272, and is provided with a warning pattern. When the state indication light 1272 is turned on, the warning pattern can be highlighted. The arrangement of the warning sheet 1271 can add a warning sign and facilitate the beautification of the visual effect of the driving module for the liquid-stopping clamp 120.

In an embodiment, a third detection member can also be installed inside the main body 110. When the infusion tube 300 is in the installation position for the infusion tube, the infusion tube 300 triggers the third detection member to output second in-position information. By arranging the third detection member, it can be determined whether the infusion tube 300 is installed in position.

In an embodiment, the processor can also only receive the second in-position information and control the electric driving mechanism 113 to switch the movable mechanism 122 from the liquid-stopping working position to the connecting working position, according to the second in-position information. Alternatively, the processor can also receive the first in-position information and the second in-position information, and control the electric driving mechanism 113 to switch the movable mechanism 122 from the liquid-stopping working position to the connecting working position, according to the first in-position information and the second in-position information.

It can be understood that regardless of whether the driving force electrically or mechanically drives the movable mechanism 122 to switch from the liquid-stopping working position to the connecting working position, the processor can receive the first and/or second in-position information. However, for the infusion pump 100 driven by mechanical driving force, after receiving the first in-position information and/or the second in-position information, there is no such step to control the electric driving mechanism 113 to drive the movable mechanism 122 from the liquid-stopping working position to the connecting working position, and it is not possible to trigger the switching of the working positions by using the first/second position information.

Please refer to FIGS. 4, 5, 8 to 10. In one embodiment, the driving module for the liquid-stopping clamp 120 further includes a second detection member 125. When the movable mechanism 122 is in the connecting working position, the movable mechanism 122 triggers the second detection member 125 to output connecting information. That is to say, by arranging the second detection member 125, it can be determined whether the movable mechanism 122 is in position, and thus whether the infusion tube 300 is connected, can be determined.

There are various ways for the movable mechanism 122 to trigger the second detection member 125 to output the connecting information, such as referring to FIGS. 4, 5, 8 to 10. In one embodiment, the second detection member 125 is a second optocoupler, which includes a second transmitting terminal 1251 and a second receiving terminal 1252 which are spaced oppositely. The movable mechanism 122 is in rotational connection with the box body 121, which includes an abutting member 1221 and a second triggering member 1223 which is connected with the abutting member 1221. When the movable mechanism 122 is at the liquid-stopping working position, the abutting member 1221 abuts against the liquid-stopping clamp 200, and the second triggering member 1223 is located at the interval between the second transmitting terminal 1251 and the second receiving terminal 1252. At this time, the second triggering member 1223 disconnects a signal transmission path between the second transmitting terminal 1251 and the second receiving terminal 1252. The second receiving terminal 1252 is unable to receive a signal transmitted by the second transmitting terminal 1251. When the movable mechanism 122 is rotated to switch from the liquid-stopping working position to the connecting working position, the second triggering member 1223 moves out of the interval between the second transmitting terminal 1251 and the second receiving terminal 1252, and the signal transmission path between the second transmitting terminal 1251 and the second receiving terminal 1252 is conducted. The second receiving terminal 1252 receives the signal transmitted by the second transmitting terminal 1251, thereby enabling the second optocoupler to output the connecting information.

In some embodiments, it is also possible that when the movable mechanism 122 is at the liquid-stopping working position, the second triggering member 1223 is located outside the second optocoupler, and when the movable mechanism 122 is rotated to switch from the liquid-stopping working position to the connecting working position, the second triggering member 1223 moves into the interval between the second transmitting terminal 1251 and the second receiving terminal 1252.

It can be understood that the second detection member 125 is not limited to a second optocoupler. In some embodiments, the second detection member 125 can also be a Hall sensor, button switch, optical proximity sensor, pressure sensor, or ultrasonic sensor, etc. In some embodiments, the second triggering member 1223 can also be an independent component which is separated from the movable mechanism 122. For example, the second triggering member 1223 can be movably arranged inside the box body 121. When the movable mechanism 122 is switched from the liquid-stopping working position to the connecting working position, the movable mechanism 122 pushes the second triggering member 1223 to trigger the second detection member 125 to output connecting information.

In some embodiments, the second triggering member 1223 can also trigger the second detection member 125 to output the connecting information through a rectilinear movement.

In an embodiment, the processor can be configured to receive the connecting information and control the infusion pump 100 to enter the infusion phase. In other words, the processor can start the infusion pump 100 after determining that the infusion tube 300 is in a connecting state, thereby improving the safety of the infusion pump 100.

It can be understood that the processor can receive the connecting information, no matter the driving force for switching the moveable mechanism 122 from the liquid-stopping working position to the connecting working position, is electric or mechanical driving force.

The embodiment of this disclosure also provides an infusion system, as shown in FIGS. 1 to 15. The infusion system includes a liquid-stopping clamp 200, an infusion tube 300, and an infusion pump 100 as described in the above embodiment. The liquid-stopping clamp 200 is sleeved outside the infusion tube 300 and is detachably connected with the driving module for the liquid-stopping clamp 120. In other words, the liquid-stopping clamp 200 and the driving module for the liquid-stopping clamp 120 are separate structures. The liquid-stopping clamp 200 can be used as a consumable material, and used in cooperation with the infusion tube 300. For example, each infusion tube 300 can be provided with one liquid-stopping clamp 200. After the infusion is completed, the liquid-stopping clamp 200 can be discarded along with the infusion tube 300, or can also be reused.

It can be understood that the infusion pump 100 can also have other structural forms. For example, in one embodiment, the infusion pump 100 can be used in cooperation with the infusion tube 300 and a liquid-stopping component capable of being disassembled. The liquid-stopping component can be the liquid-stopping clamp 200 described in the above embodiment, or can be in other structural forms. It should be noted that, the liquid-stopping component capable of being disassembled mentioned here mainly refers to a liquid-stopping component that can be disassembled with the infusion pump 100, and the liquid-stopping component can be disassembled with the infusion tube 300 or fixed to the infusion tube 300. Specifically, along the installation position for the infusion tube, the infusion pump 100 is provided with an area for pressing the infusion tube, at least one pressure detection area, and at least one bubble detection area. When the infusion tube 300 is loaded individually (there are no other liquid-stopping structures at the infusion pump 100) at the installation position for the infusion tube, a section of the infusion tube 300, which section is located upstream or downstream of the area for pressing the infusion tube, is in a connecting state. When the infusion tube 300 is combined with a liquid-stopping component capable of being disassembled and then loaded at the installation position for the infusion tube (i.e., in this embodiment, both the infusion tube 300 and the liquid-stopping component are loaded at the installation position for the infusion tube, and no separate installation position is arranged for the liquid-stopping component), the infusion pump 100 drives the liquid-stopping component for a shape adjustment. The shape adjustment here mainly refers to an adjustment for the shape of the liquid-stopping component when it cooperates with the infusion tube 300. For example, the liquid-stopping component can be adjusted from a clamp shape in which it clamps the infusion tube 300 for stopping the liquid inside the infusion tube 300, to a release shape in which it releases the infusion tube 300 for connecting the infusion tube 300. Optionally, the liquid-stopping component can also be adjusted from the release shape to the clamp shape. This allows the section of the infusion tube 300, which section is located upstream or downstream of the area for pressing the infusion tube, to switch between the connecting state and the disconnecting state. In other words, depending on the loading position of the liquid-stopping component, the liquid-stopping component can switch the section of the infusion tube 300, which section is located upstream of the area for pressing the infusion tube, between the connecting state and the disconnecting state, as well as switch the section of the infusion tube 300, which section is located downstream of the area for pressing the infusion tube, between the connecting state and the disconnecting state.

The shape of the liquid-stopping component can be adjusted by the driving force of the electric driving mechanism 113. For example, the electric driving mechanism 113 can drive a movable component of the infusion pump 100 according to a control instruction to adjust the shape of the liquid-stopping component. The shape of liquid-stopping component can also be adjusted by the mechanical driving force, such as based on the opening and closing of the pump door 130.

In an embodiment, when the liquid-stopping component is loaded at the installation position for the infusion tube, the liquid-stopping component overlaps the movable component in the perpendicular direction, that is, the movable component can adjust the shape of the liquid-stopping component in the perpendicular direction.

In an embodiment, when the liquid-stopping component which carries the infusion tube 300, is loaded at the installation position for the infusion tube, the infusion pump 100 drives the liquid-stopping component to a liquid-stopping position, so as to enable a section of the infusion tube 300, which section is located upstream or downstream of the area for pressing the infusion tube, to be in a disconnecting state, and enable a section of the infusion tube 300, which section is located inside the area for pressing the infusion tube, to be in a connecting state. When pump door 130 is closed, the pump door 130 and a pump body of the main body 110 jointly press the infusion tube 300, so as to enable the section of the infusion tube 300, which section is located inside the area for pressing the infusion tube, to be in a disconnecting state, wherein at any time point after the pump door 130 is closed, the liquid-stopping component can be switched to a connecting position, so as to enable the section of the infusion tube 300, which section is located upstream or downstream of the area for pressing the infusion tube, to be in a connecting state. When the pump door 130 is triggered to open, the liquid-stopping component is firstly driven to switch to a liquid-stopping position, so as to enable the section of the infusion tube 300, which section is located upstream or downstream of the area for pressing the infusion tube, to be in a disconnecting state, and then the pump door 130 is opened to enable the section of the infusion tube, which section is located inside the area for pressing the infusion tube, to return to the connecting state. That is to say, only when pump door 130 is in the closed state, the section of the infusion tube, which section is upstream or downstream of the area for pressing the infusion tube, can be connected. When pump door 130 is in the open state, the section of the infusion tube 300, which section is upstream or downstream of the area for pressing the infusion tube, remains in the disconnecting state, thereby improving the liquid stopping effect.

In an embodiment, the liquid-stopping component capable of being disassembled further includes a locking mechanism, and the pump door 130 is provided with an unlocking mechanism. When the pump door 130 is closed, the unlocking mechanism unlocks the locking mechanism. That is to say, before the pump door 130 is closed, the liquid-stopping component is in a locked state, and the infusion tube 300 is clamped by the liquid-stopping component. At this time, the movable component 122 of the infusion pump 100 cannot drive the liquid-stopping component to release the infusion tube 300. After the pump door 130 is closed, the unlocking mechanism unlocks the locking mechanism, and the unlocked liquid-stopping component can clamp or release the infusion tube 300 under the drive of the movable component 122.

Please refer to FIGS. 1, 2, and 13 to 15. In one embodiment, the locking mechanism can be a combination of the first clamp member 240 and the second clamp member 250 shown in FIGS. 13 to 15, and the unlocking mechanism can be the pushing member 130a shown in FIG. 2. In other embodiments, the locking mechanism and unlocking mechanism can also be other structural forms, as long as it is ensured that the unlocking mechanism can trigger the locking mechanism to unlock after the pump door 130 is closed.

In an embodiment, please refer to FIG. 1. The infusion pump 100 has a split-type liquid-stopping component, which includes a liquid-stopping fixed end 141 and a liquid-stopping movable end 142 which are used in cooperation with each other. The liquid-stopping fixed end 141 is arranged at the main body 110, and the liquid-stopping movable end 142 is arranged at the infusion tube 300. The split-type liquid-stopping component 140 can be the liquid-stopping clamp 200 described in the above embodiment, or other structural forms. The liquid-stopping fixed end 141 can be the driving module for the liquid-stopping clamp 120 described in the above embodiment, or can be other structural forms. Before the infusion is started, the liquid-stopping movable end 142 is correctly combined with the liquid-stopping fixed end 141, and the liquid-stopping movable end 142 remains in a clamp state to disconnect a path for a liquid flow inside the infusion tube 300. When the infusion is started, the liquid-stopping fixed end 141 drives the liquid-stopping movable end 142 to switch to a release state, so as to connect the path for the liquid flow inside the infusion tube 300. When the infusion is completed and an instruction for opening pump door is received or the pump door 130 is detected to be opened, the liquid-stopping fixed end 141 drives the liquid-stopping movable end 142 to switch to the clamp state, so as to disconnect the path for the liquid flow inside the infusion tube 300 again.

The position of the movable component 122 of the liquid-stopping fixed end 141 can be adjusted through the electric driving mechanism 113 or under the action of mechanical driving force.

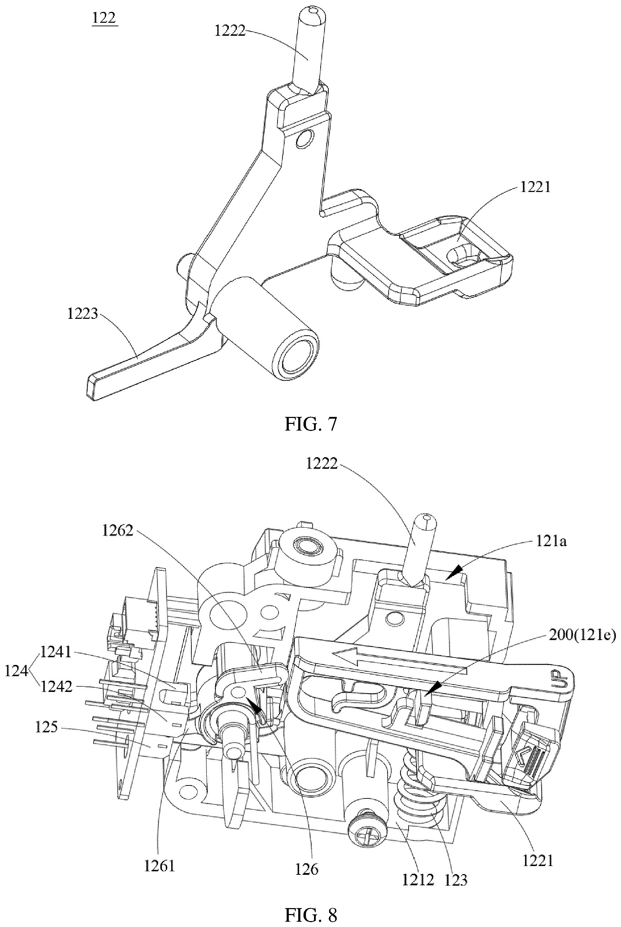

In an embodiment, please refer to FIGS. 5 and 8. The liquid-stopping fixed end 141 is provided with an insertion groove 121e, which is configured to accommodate the liquid-stopping movable end 142. The movable component 122 of the liquid-stopping fixed end 141 and the liquid-stopping movable end 142 are overlapped in the perpendicular direction. That is to say, the liquid-stopping movable end 142 can clamp or release the infusion tube 300 in a perpendicular direction, thereby facilitating the control of the liquid-stopping movable end 142 by the movable component 122 of the liquid-stopping fixed end 141.

The embodiment of this disclosure also provides a control method for an infusion pump 100, which is applied to the infusion pump 100. Please refer to FIGS. 1 to 15. The infusion pump 100 is used in cooperation with a liquid-stopping clamp 200 which is sleeved outside an infusion tube 300. The infusion pump 100 further includes a main body 110, a pump door 130, and a driving module for the liquid-stopping clamp 120. The main body 110 includes a surface frame 111, a pump piece, and a pump body, wherein the surface frame is provided with an installation position for the liquid-stopping clamp and an installation position for the infusion tube. The pump door 130 is in movable connection with the main body 110 to selectively avoid or block the installation position for the liquid-stopping clamp and the installation position for the infusion tube. The driving module for the liquid-stopping clamp 120 includes a movable mechanism 122. When the liquid-stopping clamp 200 is loaded at the installation position for the liquid-stopping clamp, the liquid-stopping clamp 200 abuts against the movable mechanism 122.

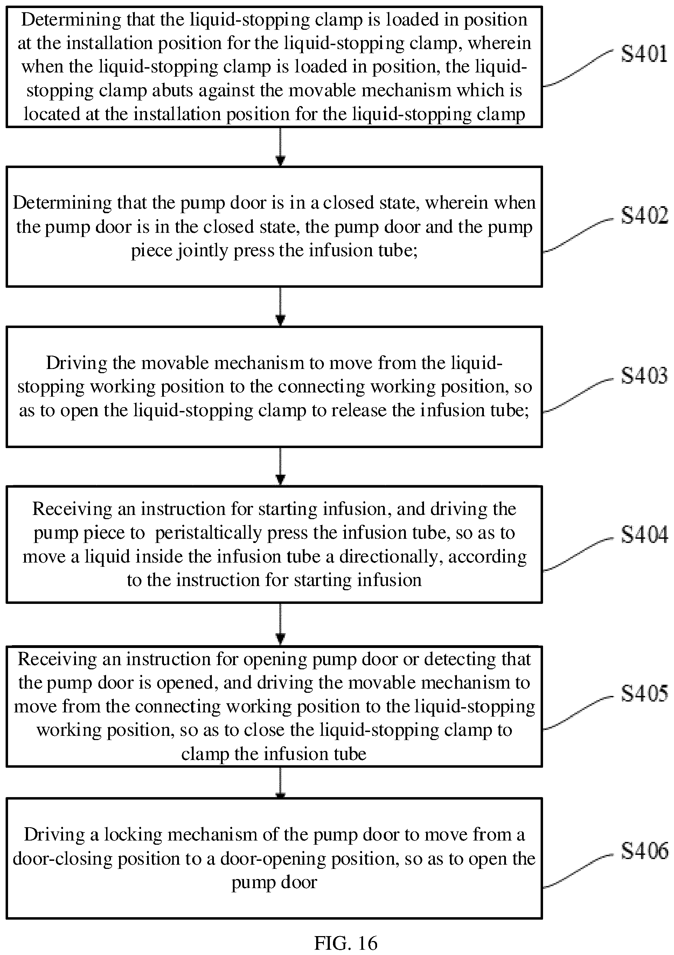

Referring FIG.16, the method include following steps.

In step 401, it is determined that the liquid-stopping clamp 200 is loaded in position at the installation position for the liquid-stopping clamp. Wherein when the liquid-stopping clamp 200 is loaded in position, the liquid-stopping clamp 200 abuts against the movable mechanism 122, which is located at a liquid-stopping working position.

That is to say, the infusion tube 300 is in a liquid-stopping state at this time.

In step 402, it is determined that the pump door 130 is in a closed state. Wherein when the pump door is 130 in the closed state, the pump door 130 and the pump piece jointly press the infusion tube 130.

Wherein, steps 401 and 402 are not defined in any order, and any one of steps 401 and 402 can be executed first, the other one can be executed later, or they can be executed synchronously.

In step 403, the movable mechanism 122 is driven to switch from the liquid-stopping working position to a connecting working position, so as to enable the liquid-stopping clamp 200 to release the infusion tube 300.

That is to say, after the pump door 130 is closed, the movable mechanism 122 can only switch from the liquid-stopping working position to the connecting working position.

In step 404, an instruction for starting infusion is received and the pump piece is driven to peristaltically press the infusion tube, so as to move a liquid inside the infusion tube directionally, according to the instruction for starting infusion.

That is to say, the infusion pump 100 can perform normal infusion at this time.

In step 405, an instruction for opening pump door is received, or it is detected that the pump door 130 is opened, the movable mechanism 122 is driven to switch from the connecting working position to the liquid-stopping working position, so as to close the liquid-stopping clamp 200 to clamp the infusion tube 300.

That is to say, after the pump door 130 is opened, the movable mechanism 122 needs to move to the liquid stopping position to stop the liquid inside the infusion tube 300 from flowing.

In step 406, a locking mechanism of the pump door 130 is driven to switch from a door-closing position to a door-opening position, so as to open the pump door 130.

The driving force for the locking mechanism of pump door 130 to switch from the door-closing position to a door-opening position can be either electric or mechanical. For example, the user can manually open pump door 130.

That is, when the pump door 130 is in the closed state, the movable mechanism 122 can only switch from the liquid-stopping working position to the connecting working position, so as to connect the infusion tube 300. When pump door 130 is in the open state, the movable mechanism 122 remains at the liquid-stopping working position to keep the infusion tube 300 in the liquid-stopping state. While facilitating the liquid stopping, occurrence in which the user fails to close the liquid-stopping clamp 200 in a timely manner or even forgets to close the liquid-stopping clamp 200, can be avoided.

It should be noted that this control method is mainly aimed at the infusion pump 100 driven by the electric driving mechanism 113, and the relevant control can be achieved by the processor.

In an embodiment, after determining that pump door 130 is in a closed state and before driving the movable mechanism 122 to switch from the liquid-stopping working position to the connecting working position, the method further includes:
controlling the pump piece to peristaltically press the infusion tube 300;
detecting a change of a pressure of the infusion tube 300;
driving the movable mechanism 122 to switch from the liquid-stopping working position to the connecting working position, if the change of the pressure of the infusion tube 300 is determined to satisfy a preset condition;
outputting alarm information if the change of the pressure of the infusion tube 300 is determined to not satisfy the preset condition.

Specifically, the pump piece can be driven to first press the infusion tube in the direction of infusion (a forward rotation of the pump piece), and then press the infusion tube in the opposite direction of infusion (a reverse rotation of the pump piece). This approach can prevent the generation of discrete dosage after the liquid-stopping clamp 200 is released. During this process, the change of the pressure of the infusion tube is detected by a pressure sensor which is arranged between the area for pressing the infusion tube and the liquid-stopping clamp. In the case of no breakage or leakage of the infusion tube, the pressure of the infusion tube in this section increases to a preset first pressure threshold when the pump piece rotates forward, and then gradually decreases when the pump piece rotates reversely. If the infusion tube breaks and leaks, the pressure of the infusion tube in this section cannot increase to the preset first pressure threshold when the pump piece rotates forward. Accordingly, at this point, the change of the pressure of the infusion tube in this section can be configured to determine whether the infusion tube leaks. There is another method, where the pump piece can be driven to first press the infusion tube in the opposite direction of the infusion (a reverse rotation of the pump piece), and then press the infusion tube in the direction of the infusion (a forward rotation of the pump piece). This method can prevent the blood withdrawal caused by the connection of the liquid-stopping clamp. During this process, if there is no breakage or leakage of the infusion tube, the pressure of the infusion tube in this section decreases to a preset second pressure threshold when the pump piece rotates reversely, then gradually increases when the pump piece rotates forward. If the infusion tube breaks and leaks, the pressure of the infusion tube in this section cannot decrease to the preset second pressure threshold when the pump piece rotates reversely. Through the leak detection on infusion tube 300, the safety of infusion pump 100 can be improved.

In an embodiment, after determining that the pump door 130 is closed and before driving the movable mechanism 122 to switch from the liquid-stopping working position to the connecting working position, the liquid-stopping clamp 200 abuts against by the movable mechanism 122 to clamp the infusion tube 300 for disconnecting the path for the liquid flow inside the infusion tube 300. That is to say, as long as the movable mechanism 122 is at the liquid-stopping working position, the liquid-stopping clamp 200 can continuously clamp the infusion tube 300 to maintain the infusion tube 300 in the liquid-stopping state.

In an embodiment, the infusion pump 100 further includes a sensor for sensing a state of the liquid-stopping clamp, which sensor is configured to determine whether the liquid-stopping clamp 200 is in a clamp state for stopping the liquid inside the infusion tube 300 or a release state for connecting the infusion tube 300. The method further includes following steps:
determining a state of the liquid-stopping clamp 200;
outputting alarm information, if the state of the liquid-stopping clamp 200 is determined to not match a preset state of the liquid-stopping clamp in a current phase, wherein multiple preset states of the liquid-stopping clamp exist, wherein the multiple preset states of the liquid-stopping clamp respectively correspond to different infusion phases.

In the clamp state, the liquid-stopping clamp 200 is closed to clamp the infusion tube 300 to stop the liquid. In the release state, the liquid-stopping clamp 200 releases the infusion tube 300 to connect the infusion tube 300. Different infusion phases can include a preparation stage before starting the infusion, start stage of the infusion, and a tube removal stage after the infusion is completed. In the preparation stage and tube removal stage, the preset state of the liquid-stopping clamp is the clamp state. If the detected state of the liquid-stopping clamp is the release state, the alarm information is outputted. During the start stage of the infusion, the preset state of the liquid-stopping clamp is the release state. If the detected state of the liquid-stopping clamp is the clamp state, the alarm information is outputted similarly. Therefore, the infusion pump 100 can detect the state of the liquid-stopping clamp 200 or the state of the driving module for the liquid-stopping clamp 120 through the signal of the sensor, which can improve the safety of the use of the infusion tube 300.

In an embodiment, if the detected state of the liquid-stopping clamp 200 does not match the current working position of the movable mechanism 122, the alarm information is output. The current working position of movable mechanism 122 includes a connecting working position or a liquid-stopping working position. That is to say, the sensor for sensing the state of the liquid-stopping clamp, can indirectly obtain the state of the liquid-stopping clamp 200 by detecting the current working position of the movable mechanism 122. The sensor for sensing the state of the liquid-stopping clamp can be the second detection member 125 mentioned above or other detection members.

In an embodiment, the infusion pump 100 further includes a sensor for sensing whether the infusion tube is in position, which sensor is configured to determine whether the infusion tube 100 is loaded at the installation position for the infusion tube. After determining that pump door 130 is closed and before driving the movable mechanism 122 to switch from the liquid-stopping working position to the connecting working position, the method further includes following steps:
determining whether the infusion tube 100 is correctly loaded at the installation position for the infusion tube;
driving the movable mechanism 122 to switch from the liquid-stopping working position to the connecting working position, if the infusion tube 100 is determined to be correctly loaded at the installation position for the infusion tube;
outputting alarm information, if the infusion tube 100 is determined to be incorrectly loaded at the installation position for the infusion tube.

The sensor for sensing whether the infusion tube is in position, can be the three detection members mentioned above, or other detection members.

When the infusion pump 100 adopts both the method of detecting the state of the liquid-stopping clamp 200 mentioned above and the method of detecting whether the infusion tube 100 is loaded at the installation position for the infusion tube, it is equivalent to further verifying the state of the liquid-stopping clamp 200, the driving module for the liquid-stopping clamp 120, and the infusion tube 100, which can greatly improve the safety of the use of the infusion tube 300.

The various embodiments/embodiments provided in this disclosure can be combined with each other without conflicts.

The above is only a preferred embodiment of this disclosure and is not intended to limit it. For those skilled in the art, this disclosure may have various modifications and changes. Any modifications, equivalent replacements, improvements, etc. made within the spirit and principles of this disclosure are included within the scope of protection of this disclosure.

## Claims

1. An infusion pump, which is used in cooperation with a liquid-stopping clamp which is sleeved outside an infusion tube; **characterized in that**, the infusion pump comprises:
a main body, which comprises a surface frame and a pump body, wherein the surface frame is provided with an installation position for the liquid-stopping clamp and an installation position for the infusion tube;
a pump door, which is in movable connection with the main body; and
a driving module for the liquid-stopping clamp, which module is arranged at the main body and comprises a movable mechanism;
wherein the liquid-stopping clamp is configured to abut against the movable mechanism which is located at a liquid-stopping working position, so as to close the liquid-stopping clamp to clamp the infusion tube for disconnecting a path for a liquid flow inside the infusion tube, when the liquid-stopping clamp is loaded at the installation position for the liquid-stopping clamp;
the liquid-stopping clamp is further configured to release the infusion tube for connecting the path for the liquid flow inside the infusion tube, when the movable mechanism is switched from the liquid-stopping working position to a connecting working position under a driving force;
the liquid-stopping clamp is further closed to clamp the infusion tube for disconnecting the path for the liquid flow inside the infusion tube, when the movable mechanism is switched from the connecting working position to the liquid-stopping working position under a driving force.

2. The infusion pump according to claim 1, **characterized in that**, the movable mechanism is configured to rotate or move rectilinearly, relative to the surface frame, when the movable mechanism is switched between the connecting working position and the liquid-stopping working position.

3. The infusion pump according to claim 1, **characterized in that**, the main body further comprises an electric driving mechanism, which is configured to provide an electric driving force for driving the movable mechanism to switch from the liquid-stopping working position to the connecting working position, or for driving the movable mechanism to switch from the connecting working position to the liquid-stopping working position; or
the driving module for the liquid-stopping clamp further comprises an elastic member, which is configured to provide an elastic force for driving the movable mechanism to switch from the liquid-stopping working position to the connecting working position, or for driving the movable mechanism to switch from the connecting working position to the liquid-stopping working position; or
the driving module for the liquid-stopping clamp further comprises an electric driving mechanism and an elastic member, wherein the electric driving mechanism is configured to provide an electric driving force for driving the movable mechanism to switch from the liquid-stopping working position to the connecting working position, the elastic member is configured to provide an elastic force for driving the movable mechanism to switch from the connecting working position to the liquid-stopping working position; or
the driving module for the liquid-stopping clamp further comprises an electric driving mechanism and an elastic member, wherein the electric driving mechanism is configured to provide an electric driving force for driving the movable mechanism to switch from the connecting working position to the liquid-stopping working position, the elastic member is configured to provide an elastic force for driving the movable mechanism to switch from the liquid-stopping working position to the connecting working position.

4. The infusion pump according to claim 1, **characterized in that**, the liquid-stopping clamp is further configured to abut against the movable mechanism which is located at the liquid-stopping working position, so as to clamp the infusion tube for disconnecting the path for the liquid flow inside the infusion tube, when the liquid-stopping clamp is loaded at the installation position for the liquid-stopping clamp, the pump door is closed, and the movable mechanism is located at the liquid-stopping working position.

5. The infusion pump according to claim 4, **characterized in that**, the movable mechanism is switched from the connecting working position to the liquid-stopping working position under the driving force, so as to close the liquid-stopping clamp to clamp the infusion tube for disconnecting the path for the liquid flow inside the infusion tube, when the movable mechanism is located at the connecting working position and the pump door is triggered to be opened.

6. The infusion pump according to claim 1, **characterized in that**, the liquid-stopping clamp comprises a first clamp arm, a second clamp arm, a first clamp member, and a second clamp member; wherein the first clamp arm and the second clamp arm are connected with each other, the first clamp member is arranged at the first clamp arm, and the second clamp member is arranged at the second clamp arm;
the pump door comprises a pushing member; wherein the first clamp member is in clamp connection with the second clamp member, when the liquid-stopping clamp is loaded at the installation position for the liquid-stopping clamp and abuts against the movable mechanism, which is located at the liquid-stopping working position; wherein the pushing member is configured to push at least one of the first clamp member and the second clamp member, so as to separate the first clamp member from the second clamp member, when the pump door is closed;
the liquid-stopping clamp is further configured to release the infusion tube for connecting the path for the liquid flow inside the infusion tube, when the first clamp member and the second clamp member are separated and the movable mechanism is switched from the liquid-stopping working position to the connecting working position under the driving force.

7. The infusion pump according to claim 6, **characterized in that**, the liquid-stopping clamp is closed to clamp the infusion tube for disconnecting the path for the liquid flow inside the infusion tube, when the movable mechanism is switched from the connecting working position to the liquid-stopping working position under the driving force; and
the first clamp member and the second clamp member are reconnected via clamping under their own elastic forces, when the pump door is opened and the pushing member is separated from the liquid-stopping clamp.

8. A control method for an infusion pump, which method is applied to the infusion pump, wherein the infusion pump is used in cooperation with a liquid-stopping clamp which is sleeved outside an infusion tube; the infusion pump comprising: a main body, a pump door, a driving module for the liquid-stopping clamp, a sensor for sensing whether the liquid-stopping clamp is in position, and a sensor for sensing a state of the pump door;
wherein the main body comprises a surface frame, a pump piece, and a pump body, wherein the surface frame is provided with an installation position for the liquid-stopping clamp and an installation position for the infusion tube; the pump door is in movable connection with the main body; the driving module for the liquid-stopping clamp comprises a movable mechanism;
**characterized in that**, the control method comprises:
determining that the liquid-stopping clamp is loaded in position at the installation position for the liquid-stopping clamp, wherein the liquid-stopping clamp is configured to abut against the movable mechanism which is located at a liquid-stopping working position, when the liquid-stopping clamp is loaded in position;
determining that the pump door is in a closed state, wherein the pump door and the pump piece are configured to jointly press the infusion tube, when the pump door is in the closed state;
driving the movable mechanism to switch from the liquid-stopping working position to a connecting working position, so as to open the liquid-stopping clamp to release the infusion tube;
receiving an instruction for starting infusion, and driving the pump piece to peristaltically press the infusion tube, so as to move a liquid inside the infusion tube directionally, according to the instruction for starting infusion;
receiving an instruction for opening pump door or detecting that the pump door is opened, and driving the movable mechanism to switch from the connecting working position to the liquid-stopping working position, so as to close the liquid-stopping clamp to clamp the infusion tube; and
driving a locking mechanism of the pump door to switch from a door-closing position to a door-opening position, so as to open the pump door.

9. The control method according to claim 8, **characterized in that**, after determining that the pump door is in a closed state, and before driving the movable mechanism to switch from the liquid-stopping working position to a connecting working position, the control method further comprises:
controlling the pump piece to peristaltically press the infusion tube;
detecting a change of a pressure of the infusion tube;
driving the movable mechanism to switch from the liquid-stopping working position to the connecting working position, if the change of the pressure of the infusion tube is determined to satisfy a preset condition; and
outputting alarm information, if the change of the pressure of the infusion tube is determined to not satisfy the preset condition.

10. The control method according to claim 8, **characterized in that**, after determining that the pump door is in a closed state, and before driving the movable mechanism to switch from the liquid-stopping working position to a connecting working position, the liquid-stopping clamp is further configured to abut against the movable mechanism, so as to clamp the infusion tube for disconnecting a path for a flow of the liquid inside the infusion tube.

11. The control method according to claim 8, **characterized in that**, the infusion pump further comprises a sensor for sensing a state of the liquid-stopping clamp, which sensor is configured to determine whether the liquid-stopping clamp is in a clamp state for stopping the liquid inside the infusion tube or in a release state for connecting the infusion tube;
wherein the control method further comprises:
determining a state of the liquid-stopping clamp; and
outputting alarm information, if the state of the liquid-stopping clamp is determined to not match a preset state of the liquid-stopping clamp in a current infusion phase, wherein multiple preset states of the liquid-stopping clamp exist, wherein the multiple preset states of the liquid-stopping clamp respectively correspond to different infusion phases.

12. The control method according to claim 8, **characterized in that**, the infusion pump further comprises a sensor for sensing a state of the liquid-stopping clamp, which is configured to determine whether the liquid-stopping clamp is in a clamp state for stopping the liquid inside the infusion tube or a release state for connecting the infusion tube;
wherein the control method further comprises:
determining a state of the liquid-stopping clamp; and
outputting alarm information, if the state of the liquid-stopping clamp is determined to not match a current working position of the movable mechanism, wherein said working position of the movable mechanism comprises the connecting working position and the liquid-stopping working position.

13. The control method according to claim 8, **characterized in that**, the infusion pump further comprises a sensor for sensing whether the infusion tube is in position, which sensor is configured to determine whether the infusion tube is loaded at the installation position for the infusion tube;
wherein after determining that the pump door is in a closed state, and before driving the movable mechanism to switch from the liquid-stopping working position to a connecting working position, the control method further comprises:
determining whether the infusion tube is correctly loaded at the installation position for the infusion tube;
driving the movable mechanism to switch from the liquid-stopping working position to the connecting working position, if the infusion tube is determined to be correctly loaded at the installation position for the infusion tube; and
outputting alarm information, if the infusion tube is determined to be incorrectly loaded at the installation position for the infusion tube.

14. An infusion pump, which is used in cooperation with a liquid-stopping clamp which is sleeved outside an infusion tube; **characterized in that**, the infusion pump comprises:
a main body, which comprises a surface frame, a pump piece, and an electric driving mechanism, wherein the surface frame is provided with an installation position for the liquid-stopping clamp and an installation position for the infusion tube;
a pump door, which is in movable connection with the main body; and
a driving module for the liquid-stopping clamp, which module is arranged at the main body and comprises a box body and a movable mechanism which is rotatably arranged inside the box body, wherein the installation position for the liquid-stopping clamp is connected with the box body;
wherein the liquid-stopping clamp is configured to abut against the movable mechanism which is located at a liquid-stopping working position, so as to close the liquid-stopping clamp to clamp the infusion tube for disconnecting a path for a liquid flow inside the infusion tube, when the liquid-stopping clamp is loaded at the installation position for the liquid-stopping clamp and the pump door is closed; wherein a direction for the liquid-stopping clamp abutting the movable mechanism is perpendicular to a direction for placing the infusion tube;
the liquid-stopping clamp is further configured to release the infusion tube for connecting the path for the liquid flow inside the infusion tube, when the electric driving mechanism drives the movable mechanism to rotate to a connecting working position;
the liquid-stopping clamp is further closed to clamp the infusion tube for disconnecting the path for the liquid flow inside the infusion tube, when the movable mechanism is rotated reversely from the connecting working position to the liquid-stopping working position under a driving force.

15. The infusion pump according to claim 14, **characterized in that**, the main body further comprises a guide member, the driving module for the liquid-stopping clamp further comprises an elastic member which is arranged inside the box body, one end of the elastic member is connected with the movable mechanism, the other end of the elastic member is connected with the box body, and the guide member is located outside the box body; wherein the electric driving mechanism comprises a driving motor, and a transmission member which is in drive connection with the driving motor;
the elastic member is in a first pressed state, when the liquid-stopping clamp is loaded at the installation position for the liquid-stopping clamp and the pump door is closed;
the guide member is driven, by the electric driving mechanism, to move rectilinearly towards a first direction; wherein during the movement of the guide member towards the first direction, the movable mechanism is pushed, by the guide member, to rotate in a first clock-moving direction until the connecting working position; wherein the movable mechanism presses the elastic member, during the rotation in the first clock-moving direction, until the elastic member is in a second pressed state; wherein a pressed amount of the elastic member in the first pressed state is smaller than that in the second pressed state;
the guide member is driven, by the electric driving mechanism, to move rectilinearly towards a second direction; wherein during the movement of the guide member towards the second direction, the movable mechanism is pushed, by the guide member, to rotate in a second clock-moving direction until the liquid-stopping working position, wherein the second clock-moving direction is opposite to the first clock-moving direction.

16. The infusion pump according to claim 14, **characterized in that**, the driving module for the liquid-stopping clamp further comprises a first detection member, and a first triggering member which is movably arranged inside the box body; wherein the liquid-stopping clamp is further configured to push the first triggering member to trigger the first detection member to output first in-position information, when the liquid-stopping clamp is loaded at the installation position for the liquid-stopping clamp; and/or,
the driving module for the liquid-stopping clamp comprises a second detection member, wherein the movable mechanism is configured to trigger the second detection member to output connecting information, when the movable mechanism is in the connecting working position.

17. The infusion pump according to claim 16, **characterized in that**, the first detection member is a first optocoupler, wherein the first optocoupler comprises a first transmitting terminal and a first receiving terminal which are spaced oppositely;
the first triggering member is in rotational connection with the box body, and the first triggering member comprises a first triggering part and an abutting top which is connected with the first triggering part; wherein the first triggering part is located at an interval between the first transmitting terminal and the first receiving terminal, when the first triggering member is in an initial state;
the liquid-stopping clamp is further configured to push the abutting top, so as to rotate the first triggering member to move the first triggering part out of the interval between the first transmitting terminal and the first receiving terminal, when the liquid-stopping clamp is loaded at the installation position for the liquid-stopping clamp.

18. The infusion pump according to claim 16, **characterized in that**, the second detection member is a second optocoupler, wherein the second optocoupler comprises a second transmitting terminal and a second receiving terminal which are spaced oppositely;
the movable mechanism comprises an abutting member, and a second triggering member which is connected with the abutting member; wherein the abutting member is configured to abut against the liquid-stopping clamp, and the second triggering member is located at an interval between the second transmitting terminal and the second receiving terminal, when the liquid-stopping clamp is loaded at the installation position for the liquid-stopping clamp;
the second triggering member is moved out of the interval between the second transmitting terminal and the second receiving terminal, when the movable mechanism is rotated to switch from the liquid-stopping working position to the connecting working position.

19. An infusion pump, which is used in cooperation with a liquid-stopping clamp which is sleeved outside an infusion tube, **characterized in that**, the infusion pump comprises:
a main body, which comprises a surface frame and a pump piece, wherein the surface frame is provided with an installation position for the liquid-stopping clamp and an installation position for the infusion tube;
a pump door, which is in movable connection with the main body; and
a driving module for the liquid-stopping clamp, which module is arranged at the main body and comprises a box body, and a movable mechanism and an elastic member, which are arranged inside the box body, wherein one end of the elastic member is connected with the movable mechanism, the other end of the elastic member is connected with the box body, wherein the installation position for the liquid-stopping clamp is connected with the box body;
wherein the elastic member is in a first pressed state, and the liquid-stopping clamp is configured to abut against the movable mechanism which is located at a liquid-stopping working position, so as to close the liquid-stopping clamp to clamp the infusion tube for disconnecting a path for a liquid flow inside the infusion tube, when the liquid-stopping clamp is loaded at the installation position for the liquid-stopping clamp; wherein a direction for the liquid-stopping clamp abutting the movable mechanism is perpendicular to a direction for placing the infusion tube;
the pump door is configured to push the movable mechanism to move, so as to press the elastic member, when the pump door is closed; wherein the elastic member is in a second pressed state, and the liquid-stopping clamp is further configured to release the infusion tube for connecting the path for the liquid flow inside the infusion tube, when the movable mechanism is moved to a connecting working position; wherein a pressed amount of the elastic member in the first pressed state is smaller than that in the second pressed state;
the elastic member is configured to push the movable mechanism under its own elastic force, so as to switch the movable mechanism from the connecting working position to the liquid-stopping working position, such that the liquid-stopping clamp is closed to clamp the infusion tube for disconnecting the path for the liquid flow inside the infusion tube, when the pump door is opened.

20. An infusion pump, which is used in cooperation with an infusion tube and a liquid-stopping component capable of being disassembled, **characterized in that**, the infusion pump comprises a main body and a pump door which are in movable connection with each other, one side of the main body is provided with an installation position for the infusion tube; wherein an area for pressing the infusion tube, at least one pressure detection area, and at least one bubble detection area, are arranged along the installation position for the infusion tube;
the infusion pump is configured to drive the liquid-stopping component for a shape adjustment, so as to enable a section of the infusion tube, which section is located upstream or downstream of the area for pressing the infusion tube, to switch between a connecting state and a disconnecting state, when the infusion tube is assembled with the liquid-stopping component and loaded at the installation position for the infusion tube;
the section of the infusion tube, which section is located upstream or downstream of the area for pressing the infusion tube, is in the connecting state, when the infusion tube is individually loaded at the installation position for the infusion tube.

21. The infusion pump according to claim 20, **characterized in that**, the main body comprises an electric driving mechanism;
wherein after the pump door is closed, the electric driving mechanism is configured to drive, according to a control instruction, a movable component of the infusion pump for the shape adjustment of the liquid-stopping component, so as to enable the section of the infusion tube, which section is located upstream or downstream of the area for pressing the infusion tube, to switch between the connecting state and the disconnecting state.

22. The infusion pump according to claim 21, **characterized in that**, the liquid-stopping component and the movable component are overlapped in a perpendicular direction, when the liquid-stopping component is loaded at the installation position for the infusion tube.

23. An infusion pump, **characterized in that**, comprising a main body and a pump door which are in movable connection with each other, one side of the main body is provided with an installation position for an infusion tube; wherein an area for pressing the infusion tube, at least one pressure detection area, and at least one bubble detection area, are arranged along the installation position for the infusion tube;
wherein the infusion pump further comprises a split-type liquid-stopping component, which comprises a liquid-stopping fixed end and a liquid-stopping movable end, which are used in cooperation with each other, wherein the liquid-stopping fixed end is arranged at the main body, and the liquid-stopping movable end is arranged at the infusion tube;
before starting infusion, the liquid-stopping movable end is correctly combined with the liquid-stopping fixed end, and the liquid-stopping movable end is maintained in a clamp state for disconnecting a path for a liquid flow inside the infusion tube;
when starting infusion, the liquid-stopping fixed end is configured to drive the liquid-stopping movable end to switch to a release state for connecting the path for the liquid flow inside the infusion tube;
after completing infusion, and receiving an instruction for opening pump door or detecting that the pump door is opened, the liquid-stopping fixed end is configured to drive the liquid-stopping movable end to switch to the clamp state, for disconnecting the path for the liquid flow inside the infusion tube again.

24. The infusion pump according to claim 23, **characterized in that**, the liquid-stopping fixed end is provided with an insertion groove for accommodating the liquid-stopping movable end, wherein a movable component of the liquid-stopping fixed end and the liquid-stopping movable end are overlapped and placed perpendicular to a direction for placing the infusion tube.

25. The infusion pump according to claim 23, **characterized in that**, the main body further comprises an electric driving mechanism, which is configured to adjust a position of a movable component of the liquid-stopping fixed end, so as to drive the liquid-stopping movable end to switch between the clamp state and the release state.

26. An infusion pump, **characterized in that**, comprising a main body and a pump door which are in movable connection with each other, the main body is provided with an installation position for an infusion tube; wherein an area for pressing the infusion tube, at least one pressure detection area, and at least one bubble detection area, are arranged along the installation position for the infusion tube;
the infusion pump is configured to drive a liquid-stopping component capable of being disassembled to a liquid-stopping position, so as to enable a section of the infusion tube, which section is located upstream or downstream of the area for pressing the infusion tube, to be in a disconnecting state, and to enable a section of the infusion tube, which section is located inside the area for pressing the infusion tube, to be in a connecting state, when the liquid-stopping component, which carries the infusion tube, is loaded at the installation position for the infusion tube;
when the pump door is closed, the pump door and a pump piece of the main body are configured to jointly press the infusion tube, so as to enable the section of the infusion tube, which section is located inside the area for pressing the infusion tube, to be in a disconnecting state; wherein at any time point after the pump door is closed, the liquid-stopping component is switched to a connecting position, so as to enable the section of the infusion tube, which section is located upstream or downstream of the area for pressing the infusion tube, to be in a connecting state;
when the pump door is triggered to open, the liquid-stopping component is firstly switched to the liquid-stopping position, so as to enable the section of the infusion tube, which section is located upstream or downstream of the area for pressing the infusion tube, to be in a disconnecting state, and then the pump door is opened, so as to enable the section of the infusion tube, which section is located inside the area for pressing the infusion tube, to return to a connecting state.

27. The infusion pump according to claim 26, **characterized in that**, the liquid-stopping component further comprises a locking mechanism, and the pump door is provided with an unlocking mechanism, wherein the unlocking mechanism is configured to unlock the locking mechanism, when the pump door is closed.
